# EUROPÄISCHE PATENTANMELDUNG

(11) **EP 4 814 151 A1**
(43) Veröffentlichungstag der Anmeldung: **30.09.2026**
(21) Anmeldenummer: 25167043.6
(22) Anmeldetag: 28.03.2025
(51) Int. Cl.: C22C 33/04, C21C 5/46, G16C 20/30, G16C 60/00, C21C 5/52, C21D 1/55, C21D 8/06, C21D 9/52

(54) **VERFAHREN UND SYSTEM ZUR HERSTELLUNG VON STABSTÄHLEN**

(71) Anmelder: voestalpine Stahl Donawitz GmbH, 8700 Leoben-Donawitz (AT)
(72) Erfinder: Klösch, Gerald, 8700 Leoben (AT); Karner, Peter, 8700 Leoben (AT)
(74) Vertreter: WSL Patentanwälte Partnerschaft mbB

(57) **Zusammenfassung**

Die Erfindung betrifft ein Verfahren zur Herstellung von Stabstählen mit einem auf eine Stabstahlgüte spezifizierten Härteverlaufbereich, welcher durch eine Ziellegierung sichergestellt wird, wobei zumindest ein primärmetallurgisches Aggregat zu einer ersten Schmelze führt, wobei aus der ersten Schmelze zumindest eine Analyse von Gehalten der Elemente durchgeführt wird, wobei zumindest ein Ist-Wert der Schmelzanalyse an ein Steuersystem geleitet wird, wobei zwischen Einfluss- und Kompensationselementen unterschieden wird, wobei Einflusselemente Elemente sind, welche einen Einfluss auf den Härteverlauf eines Endprodukts haben, und Kompensationselemente Elemente sind, die einen Einfluss auf den Härteverlauf haben und den Einfluss von Einflusselementen kompensieren, wobei die Größe des Einflusses des zumindest einen Ist-Werts der jeweiligen Einfluss- und Kompensationselemente auf den Härteverlauf des Endprodukts bekannt ist, wobei der zumindest eine Ist-Wert bezüglich derjenigen Einfluss- und Kompensationselemente erfasst wird, welche einen Einfluss auf den Härteverlauf des Endprodukts haben, wobei zum Härteverlauf des Endprodukts zumindest ein Kompensationselement ermittelt wird, welches den Härteverlauf einstellt, wobei eines oder mehrere Kompensationselemente in die Schmelze in einer Menge zulegiert werden, welche den Härteverlauf in einen Zielbereich bringen und den Einfluss eines oder mehrerer Einflusselemente auf den Härteverlauf kompensieren, und wobei hierfür im Steuersystem zwei Komponenten verwendet werden: ein Prognosemodell und ein Korrekturmodell, wobei mit dem Prognosemodell der Einfluss der Einfluss- und Kompensationselemente auf den Härteverlauf des Endprodukts und mit dem Korrekturmodell korrigierte Zielbereiche der Kompensationselemente berechnet werden, um den Härteverlauf in einen Zielbereich zu bringen, wobei zumindest ein Prognosemodell und/oder Korrekturmodell unter Anwendung einer multiplen linearen Regression für einen stabilen Härteverlauf erzeugt wird, wobei für die Erstellung des zumindest einen Prognosemodells und/oder Korrekturmodells zunächst ein Trainingsdatensatz basierend auf einer Ermittlung der Härtbarkeit von Stahl durch EN ISO 642 erstellt wird und hierauf basierend zumindest ein Prognosemodell und/oder Korrekturmodell für die spezifizierte Stabstahlgüte erzeugt wird, sowie ein Steuersystem zur Durchführung des Verfahrens und einen Stabstahl für Wärmebehandlungsanwendungen.

## Beschreibung

Die Erfindung betrifft ein Verfahren zur Herstellung von Stabstählen, ein System zur Herstellung von Stabstählen und einen Stabstahl für Wärmebehandlungsanwendungen.

Weltweit wird Stahl zu 71,1% (in 2023) über die Hochofen-Route erzeugt. Dabei wird Roheisen aus Eisenerz gewonnen, indem dieses zusammen mit Reduktionsmitteln wie bspw. Koks und Zuschlagstoffen im Hochofen geschmolzen wird. Das geschmolzene Roheisen wird anschließend im Konverterverfahren (z. B. mit Sauerstoff) oder im Elektrolichtbogenofen (mit Schrott als Rohstoff) zu Rohstahl weiterverarbeitet.

Primärmetallurgische Aggregate, welche bei der Herstellung von Rohstahl Anwendung finden, können die folgenden sein: BF (Hochofen, Blast Furnace), RE-Ent-S (Roheisenentschwefelung, Hot Metal Desulfurization), BOF (Sauerstoffblaskonverter, Basic Oxygen Furnace), EAF (Elektrolichtbogenofen, Electric Arc Furnace), SAF (Elektroschmelzofen, Submerged Arc Furnace), IF (Induktionsofen, Induction Furnace), Smelter (Schmelzofen), MOE (Schmelzflusselektrolyse, Metal Oxide Electrolysis), Plasmet (Plasmareduktionsmetallurgie) usw. Anschließend wird der Rohstahl in der Sekundärmetallurgie weiterbehandelt, um die Zusammensetzung und Reinheit zu verbessern. Hierbei wird der Stahl in sekundärmetallurgischen Aggregaten wie Spülstand (Rinse Station, Purging Station), Pfannenofen (Ladle Furnace), RH-Anlagen (Ruhrstahl-Heraeus-Verfahren), VD-Anlagen (Vacuum Degassing), DETEM-Anlagen, VOD-Anlagen (Vacuum Oxygen Degassing), ASEA-SKF (Sauerstoff-Vakuumbehandlung mit Rühren), CAS-Anlagen (Composition Adjustment by Sealed Argon Bubbling), RS-V-Anlagen (Rheinstahl-Siemens-Vakuum), CAS-OB-Anlagen (Composition Adjustment by Sealed Argon Bubbling - Oxygen Blowing), Kalk-Silizium Behandlung (CaSi-Injection), Magnesium-Behandlung (Magnesium Injection), VLD-Verfahren (Vacuum Ladle Degassing) etc. behandelt, wobei Zusätze (metallische sowie nicht metallische Einsatzstoffe) wie Legierungselemente und Schlackenbilder hinzugefügt werden, um die gewünschte chemische Zusammensetzung und spezifische Eigenschaften des Stahls zu erzielen.

Im Hochofen wird normalerweise kein Schrott eingesetzt, da der Prozess primär auf der Reduktion von Eisenerz basiert, um flüssiges Roheisen zu erzeugen. Schrott wird allerdings in der Stahlproduktion in anderen Prozessschritten genutzt, vor allem in Elektrolichtbogenöfen oder als Kühlmittel in Konvertern.

Das in primär metallurgischen Aggregaten erzeugte Roheisen wird anschließend in einem Sauerstoffkonverter weiterverarbeitet. In diesen Konvertern, wie dem LD-Konverter (Linz-Donawitz-Verfahren), wird flüssiges Roheisen durch Einblasen von Sauerstoff entkohlt und in Rohstahl umgewandelt. Dabei wird Schrott als Kühlmittel und zusätzliche Einsatzstoffe zugegeben. Die Zugabe von Schrott im Konverterprozess hilft, die Temperatur zu kontrollieren, da die Exothermie der Sauerstoffreaktionen die Schmelze stark erhitzt.

Ein anderer wichtiger Produktionsweg in der Stahlindustrie ist der Elektrolichtbogenofen (EAF). Hier kann bis zu 100% Schrott als Einsatzstoff verwendet werden, der durch elektrische Energie eingeschmolzen wird, um flüssigen Stahl herzustellen. Dieses Verfahren ist besonders umweltfreundlich, da zumeist die energieintensivere Reduktion nicht erforderlich ist und daher weniger CO₂-Emissionen verursacht wird als beim Hochofenprozess.

Beim Elektrolichtbogenofenverfahren wird Stahl durch elektrische Energie erzeugt. Dabei wird ein elektrischer Lichtbogen zwischen Graphitelektroden und dem Schrottmaterial im Ofen erzeugt, wodurch hohe Temperaturen (bis zu 3.500 °C im Bereich des Lichtbogens) entstehen, die den Schrott schmelzen und ihn so zu flüssigem Rohstahl verarbeiten.

Bei der Herstellung von Rohstahl werden Einsatzstoffe, wie beispielsweise Schrott, verwendet. Es wird zwischen metallischen und nichtmetallischen Einsatzstoffen unterschieden. Dies sind zum Beispiel Eisenerz, Reduktionsmittel, Schlackenbildner, Eisenschwamm in Form von direkt reduziertem Eisen (DRI) bzw. heiß brikettiertem Eisen (HBI), Desoxidationsmittel, Legierungsmittel und Schrott.

Die Verwendung von Schrott als Rohmaterial für Stahl bietet mehrere Vorteile. Das Einschmelzen von Schrott benötigt lediglich die Energie zum Erwärmen bzw. Schmelzen und, da zumeist die energieintensive Reduktion von vorgelagerten Prozessen durchgeführt wird, weniger Energie als die Primärproduktion von Rohstahl aus Eisenerz. Außerdem reduziert Schrottrecycling die CO₂-Emissionen und den Bedarf an neuen Rohstoffen, was die Umweltbelastung senkt. Da Stahl immer wieder recycelt werden kann, ist die Nutzung von Schrott ein wichtiger Beitrag zur Kreislaufwirtschaft in der Stahlindustrie.

Obwohl die Schrottverwendung im Elektrolichtbogenofen (EAF) viele Vorteile mit sich bringt, gibt es auch einige Nachteile und Herausforderungen, die berücksichtigt werden müssen.

Die erste Herausforderung bei Verwendung von Schrott ist die schwankende Schrottqualität. Der Schrott, der im EAF verwendet wird, ist oft inhomogen und kann Verunreinigungen enthalten, die sich negativ auf die Stahlqualität auswirken. Zum Beispiel können unerwünschte Elemente wie Kupfer, Molybdän, Zinn, Nickel und Chrom im Schrott vorkommen, die im Rohstahl schwer zu entfernen sind und für bestimmte Anwendungen negative Auswirkungen in der Sekundärmetallurgie nachgeschalteten Anlagen und Eigenschaften des Endproduktes haben. Verunreinigungen können den EAF-Prozess komplizierter und teurer machen, da zusätzliche Aufbereitungsschritte oder Sortierungen erforderlich sein können.

Die zweite große Herausforderung betrifft die Kontrolle der chemischen Legierungszusammensetzung. Da Schrott unterschiedliche Legierungen und Metallzusammensetzungen haben kann, kann es schwer sein, die chemische Zusammensetzung des Endprodukts präzise zu steuern. Die Zugabe von Legierungselementen im EAF wird daher komplizierter, und es sind oft zusätzliche Anpassungen nötig, um die gewünschte Stahlspezifikation zu erreichen.

Aufgrund der möglichen Verunreinigungen und der schwankenden Qualität des Schrotts ist es schwer, mit dem EAF-Verfahren besonders niedrige Gehalte (< 0,005 Gew.-%) an Elementen wie Kupfer, Zinn und Zink zu gewährleisten, die für bestimmte spezialisierte Anwendungen erforderlich sind. In solchen Fällen ist die Nutzung von Primärmaterialien (bspw. Roheisen, pig iron, flüssiges Roheisen, hot metal, Eisenschwamm, DRI, HBI) oder sehr hochwertigem, sortenreinem Schrott notwendig, was die Kosten erhöht.

Neben der chemischen Zusammensetzung, welche in einem Elektrolichtbogenofenprozess einsatzstoffbedingten Schwankungen unterliegt und deshalb oft in vorbestimmten Toleranzgrenzen gehalten wird, sind auch die Auswirkungen einzelner Elemente auf die Eigenschaften des Endprodukts zu berücksichtigen.

Insbesondere bei einer nachfolgenden Wärmebehandlung oder Umformprozessen, wie beispielsweise Walzen, werden die werkstoffkundlichen Eigenschaften des Stahls zusätzlich gesteuert.

Es sind bereits einige Lösungen bekannt, welche die unerwünschten Begleitelemente in der Legierungszusammensetzung berücksichtigen oder zusätzliche Anpassungen der Legierungselemente vorsehen.

Es ist üblich, fest definierte Zielbereiche für eine Stahlgüte festzulegen, welche den minimalen und den maximalen Wert je Legierungselement vorsehen. Für Begleitelemente, welche nicht bewusst legiert aber in den Einsatzstoffen in unbekanntem Gehalt enthalten sind, existieren Maximalgehalte, welche helfen, bei Unterschreitung dieser Gehalte die Werkstoffeigenschaften innerhalb des gewünschten Bereiches zu halten. Auf den tatsächlichen Gehalt dieser Begleitelemente wird in der Flüssigphase allerdings nicht reagiert.

Dabei ist von Nachteil, dass niedrige Maximalgehalte der Begleitelemente die Verwendung von Einsatzstoffen mit geringen Gehalten dieser Elemente erzwingen. Dies wird in erster Linie durch einen hohen Anteil an aus Primärstoffen erzeugten Einsatzstoffen erreicht.

Aus der EP 3 956 481 B1 ist ein computergestütztes Verfahren zur Überwachung des Stahlherstellungsprozesses in einem Konverter bekannt. Dabei werden verschiedene Materialien mit spezifischen Eigenschaften in den Konverter eingebracht, um flüssigen Rohstahl und Schlacke zu produzieren. Bei dem Verfahren werden zuerst die gewünschten Eigenschaften des zu produzierenden flüssigen Rohstahls und der Schlacke festgelegt. Dann werden die erforderlichen Mengen jedes Materials, die in den Konverter eingebracht werden müssen, berechnet, um die definierten Zielmerkmale des Rohstahls und der Schlacke zu erreichen. Die berechneten Materialmengen werden an den Bediener oder an automatische Ladesysteme übermittelt und der Konverter wird entsprechend beladen.

Somit wird hier das Beladen des Konverters mit verschiedenen Ausgangsstoffen beschrieben. Nachteilig ist, dass dabei lediglich die Zusammensetzung der im Konverter erzeugten Schmelze kontrolliert wird. Eine nachträgliche Einflussnahme auf die Legierungszusammensetzung wird nicht spezifiziert.

Aus der DE 10 2021 211 320 A1 ist ein Verfahren zur Steuerung und Regelung einer Produktionsanlage für Walzprodukte aus metallischen Legierungen wie Stahl, Eisen oder Aluminium bekannt. Ziel des Verfahrens ist es, den Einsatz von Rohstoffen zu optimieren und die Produktionskosten zu senken. Anfangs werden für jeden Auftrag spezifische Sollwerte für Material-, Oberflächen- und geometrische Eigenschaften des Endprodukts sowie zulässige chemische Zusammensetzungen mit definierten Toleranzbereichen festgelegt. Durch Anwendung von Prozessmodellen werden für jeden Auftrag prädiktive Ist-Werte der Produkteigenschaften berechnet und mit den Sollwerten verglichen. Nur Zusammensetzungen, die innerhalb der spezifizierten Toleranzen liegen, werden ausgewählt. Für jede ausgewählte chemische Zusammensetzung werden Kostenparameter wie Legierungs-, Einsatzstoff-, Energie-, Eisen-, Zuschlagsstoff- und CO₂-Kosten ermittelt. Diese werden in Form von Strafpunkten oder einer Straffunktion quantifiziert. Anschließend wird die Schnittmenge der zulässigen chemischen Zusammensetzungen für verschiedene Kombinationen von Produktionsaufträgen, die in einer gemeinsamen Charge erschmolzen werden sollen, ermittelt.

Nachteilig dabei ist, dass hier Toleranzbereiche für jede Zusammensetzung ermittelt werden. Eine genaue Steuerung der Zusammensetzung ist mit diesem Modell nicht möglich.

Aus der EP 4 183 498 A1 ist ein KI-basiertes Prognosemodell bekannt, mit welchem sich die mechanischen Eigenschaften aus einigen vorher gemessenen Ausganswerten, wie beispielsweise der Zusammensetzung der Schmelze, vorhersagen lassen. Um einen Einfluss auf die mechanischen Eigenschaften zu nehmen, wird als Output eine Anpassung der Herstellungsparameter, wie beispielsweise Vorwalzverhältnis, Fertigwalzverhältnis, Walzstarttemperatur, Startzeit der Kühlung, Abkühlungsrate oder Liniengeschwindigkeit vorgeschlagen.

Das Modell ist auf spezifische Produktionslinien und -prozesse optimiert. Wenn die Produktionsbedingungen oder Anforderungen schnell geändert werden müssen, kann das System Schwierigkeiten haben, sich anzupassen.

Halbzeug bezeichnet stranggegossene, massive, ggf. warmvorgewalzte Erzeugnisse und andere massive, nur warmvorgewalzte oder nur vorgeschmiedete Erzeugnisse. Dies schließt auch vorprofiliertes Halbzeug ein. Diese Erzeugnisse werden nicht aufgerollt.

Flachgewalzte Erzeugnisse sind gewalzte massive Erzeugnisse mit rechteckigem (nicht quadratischem) Querschnitt, welche nicht der Begriffsbestimmung des Halbzeugs entsprechen. Solche flachgewalzten Erzeugnisse haben eine Breite von mindestens dem Zehnfachen der Dicke, sofern diese weniger als 4,75 mm beträgt, oder eine Breite von mehr als 150 mm, sofern die Dicke 4,75 mm oder mehr, jedoch nicht mehr als die Hälfte der Breite beträgt. Diese können zu Rollen (Coils) mit übereinander liegenden Lagen aufgerollt werden. Als flachgewalzte Erzeugnisse gelten auch solche Erzeugnisse, die unmittelbar vom Walzen herrührende Oberflächenmuster, wie z. B. Rillen, Riefen, Waffelungen, Tränen, Warzen, Rauten, aufweisen oder die gelocht, perforiert, gewellt oder poliert sind, sofern sie durch diese Bearbeitungen nicht den Charakter anderweit genannter Waren erhalten haben. Flachgewalzte Erzeugnisse beliebiger Abmessungen von anderer als quadratischer oder rechteckiger Form sind wie Erzeugnisse mit einer Breite von 600 mm oder mehr einzureihen, sofern sie nicht den Charakter von Waren erhalten haben, die von anderen Positionen erfasst werden.

Walzdraht bezeichnet in Ringen regellos aufgehaspelte warmgewalzte massive Erzeugnisse mit Querschnitt in Form eines Kreises, Kreisabschnitts, Ovals, Quadrats, Rechtecks, Dreiecks oder eines anderen konvexen Vielecks (einschließlich "abgeflachte Kreise" und "modifizierte Rechtecke", bei denen zwei gegenüberliegende Seiten die Form von konvexen Bögen aufweisen, während die beiden anderen Seiten gerade, von gleicher Länge und parallel sind). Diese Erzeugnisse können vom Walzen herrührende Einschnitte, Rippen (Wülste), Vertiefungen oder Erhöhungen aufweisen (Betonarmierungsstähle).

Stabstahl bezeichnet massive Erzeugnisse, die weder den Begriffsbestimmungen von Halbzeug, flachgewalzten Erzeugnissen und Walzdraht noch der Begriffsbestimmung für Draht entsprechen. Stabstahl weist einen über die gesamte Länge gleichbleibenden Querschnitt in Form eines Kreises, Kreisabschnitts, Ovals, Quadrats, Rechtecks, Dreiecks oder anderen konvexen Vielecks (einschließlich "abgeflachte Kreise" und "modifizierte Rechtecke", bei denen zwei gegenüberliegende Seiten die Form von konvexen Bogen aufweisen, während die beiden anderen Seiten gerade, von gleicher Länge und parallel sind) auf. Stabstahl-Erzeugnisse können vom Walzen herrührende Einschnitte, Rippen (Wülste), Vertiefungen oder Erhöhungen aufweisen (Betonarmierungsstähle).

Stabstahl ist eine Unterart der sog. Metall-Halbzeuge, welche metallische Halbfertigprodukte sind. In Bezug auf ihre geometrische Form, ist der Querschnitt im Vergleich zur Länge kleiner. Somit sind diese Halbfertigprodukte als länglich (im Falle von Langprodukten) zu bezeichnen. Stabstahl bzw. Stangenmaterial ist demnach ein stabförmiges Bauteil aus Vollmaterial, welches über seine gesamte Länge einen gleichbleibenden, meist kreis-, rechteck- oder sechseckförmigen Querschnitt aufweist, welcher durch Umformen hergestellt wird. Stabstahl bildet das Ausgangsmaterial für das fertige Werkstück.

Stabstähle, welche einer nachfolgenden Wärmebehandlung wie beispielsweise Vergüten oder Einsatzhärten unterzogen werden, werden z.B. als Vergütungsstabstähle (EN ISO 683-1 Heat-treatable steels, alloy steels and free-cutting steels - Part 1: Non-alloy steels for quenching and tempering und EN ISO 683-2, Heat-treatable steels, alloy steels and free-cutting steels - part 2: Alloy steels for quenching and tempering) oder Einsatzstabstähle (EN ISO 683-3, Heat-treatable steels, alloy steels and free-steels - part 3: Case-hardening steels) oder Wälzlagerstähle (EN ISO 683-17 Heat-treated steels, alloy steels and free-cutting steels - Part 17: Ball and roller bearing steels) bezeichnet. In den meisten Fällen muss der Stabstahl für die Anwendung ein bestimmtes Härteprofil (durch minimale und maximale Härtewerte definierter Härteverlaufbereich), welches mittels eines Stirnabschreckversuchs (EN ISO 642, Steel - Hardenability test by end quenching (Jominy test)) überprüft wird, aufweisen.

Als Werkstoffe kommen hier unlegierte und niedriglegierte Edelstähle in Betracht, da diese in den meisten Fällen für ein Vergüten oder eine Oberflächenhärtung vorgesehen sind. Ein wichtiges Kriterium hierbei ist, dass der Werkstoff gleichmäßig von der jeweiligen Behandlung erfasst wird. Somit ist sowohl die genaue Einstellung der chemischen Zusammensetzung als auch die Sorgfalt in der Prozessführung wichtig, um den hohen (Produkt-)Anforderungen zu entsprechen. Diese Anforderungen äußern sich in den werkstoffkundlichen Eigenschaften, insbesondere mechanisch-technologischen Eigenschaften dieser Stähle. Dies wären zum Beispiel:
- Hohe oder eng eingeschränkte Streckgrenzen und Zähigkeiten (festgelegte Mindestwerte der Kerbschlagarbeit im vergüteten Zustand);
- Enge Härtbarkeitswerte (festgelegte Härtebänder und Einhärtungstiefen (Härteverlaufbereiche) oder Oberflächenhärten im gehärteten, vergüteten oder oberflächengehärteten Zustand);
- Hohe Reinheitsgradanforderungen (geringer Gehalt an nichtmetallischen Einschlüssen);
- Kaltumformbarkeit und Spanbarkeit;
- Schweißbarkeit (festgelegte Höchstwerte an Phosphor und Schwefel).

Als Werkstoffe kommen zum Beispiel in Betracht:
- Stähle ohne Bor (EN ISO 683-2), wie zum Beispiel 34Cr4/34CrS4, 37Cr4/37CrS4, 41Cr4/41Cr4S4, 34CrMo4/34CrMo4S4, 42CrMo4/42CrMo4S4, 50CrMo4, 41CrNiMo2/41CrNiMo2S4, 51CrV4, 36CrNiMo4, 34CrNiMo6, 30CrNiMo8;
- Stähle mit Bor (EN ISO 683-2), wie zum Beispiel 20MnB5, 30MnB5, 39MnB5, 27MnCrB5-2, 33MnCrB5-2, 39MnCrB6-2;
- Unlegierte Stähle (EN ISO 683-3), wie zum Beispiel C10E/C10R, C15E/C15R, C16E/C16R, 22Mn6;
- Legierte Stähle (EN ISO 683-3), wie zum Beispiel 17Cr3/17CrS3, 20Cr4/20CrS4, 28Cr4/28CrS4, 16MnCr5/16MnCrS5/16MnCrB5, 20MnCr5/20MnCrS5, 18CrMo4/18CrMoS4, 24CrMo4/24CrMo4S, 22CrMoS3-5, 20MoCr4/20MoCrS4, 16NiCr4/16NiCrS4, 18NiCr5-4, 17CrNi6-6, 15NiCr13, 20NiCrMo2-2/20NiCrMoS2-2, 17NiCrMo6-4/17NiCrMoS6-4, 23MnCrMo5-5-4, 18CrNiMo7-6;
- Walzlagerstähle (EN ISO 683-17), wie zum Beispiel 100Cr6, 100CrMnSi4-4, 100CrMnSi6-4, 100CrMnSi6-6, 100CrMo7, 100CrMo7-3, 100CrMo7-4, 100CrMnMoSi8-4-6, C56E2, 56Mn4, 70Mn4, 43CrMo4.

Um den Anforderungen des Zielproduktes zu genügen, können weitere Bearbeitungsschritte wie z.B. Beschichtungsprozesse notwendig werden. Anwendung finden die Produkte beispielsweise (jedoch nicht ausschließlich) in der Automobilindustrie und im Maschinenbau.

Die oben genannten engen Härtbarkeitsgrenzen von metallischen Werkstoffen können sowohl durch die Legierungselemente als auch durch eine Wärmebehandlung gesteuert werden, sodass das wärmebehandelte Bauteil seine spätere Funktion optimal erfüllen kann. Unter einer Wärmebehandlung wird nach DIN EN 10052 ein Verfahren oder eine Verfahrenskombinationen verstanden, bei denen Werkstücke oder Halbzeuge bestimmten Zeit-Temperatur-Zyklen unterliegen, wodurch es zu einer Änderung des Werkstoffgefüges und/oder der -eigenschaften kommt.

Eine solche Steuerung der Härtbarkeitsgrenzen durch gezielte Zugabe von Legierungselementen oder Wärmebehandlung ermöglich zum Beispiel die Erhöhung der Festigkeit (insbesondere Streck- bzw. Dehngrenzen) bei guter oder verbesserter (in Hinblick auf den späteren Anwendungszweck) Zähigkeit und/oder die Erhöhung der Härte und der Verschleißbeständigkeit im gesamten Querschnitt bzw. an der Oberfläche.

Aufgabe der Erfindung ist es, ein Verfahren zur Herstellung von Stabstählen zur Verfügung zu stellen, welches eine gezielte Steuerung der Härtbarkeitsgrenzen auch bei größeren Schwankungen der Begleitelemente ermöglicht.

Die Aufgabe wird mit einem Verfahren mit den Merkmalen des Anspruchs 1 gelöst.

Vorteilhafte Weiterbildungen sind in den Unteransprüchen gekennzeichnet.

Ferner ist es Aufgabe der Erfindung, ein System zur Herstellung von Stabstählen zur Verfügung zu stellen, welches eine gezielte Steuerung des Härteverlaufs innerhalb der Härtbarkeitsgrenzen auch bei größeren Schwankungen der Begleitelemente ermöglicht.

Diese Aufgabe wird mit einem Steuersystem mit den Merkmalen des Anspruchs 22 gelöst.

Vorteilhafte Weiterbildungen sind in den Unteransprüchen gekennzeichnet.

Es ist auch Aufgabe der Erfindung, einen Stabstahl für Wärmebehandlungsanwendungen zur Verfügung zu stellen.

Diese Aufgabe wird mit einem Stabstahl mit den Merkmalen des Anspruchs 28 gelöst.

Es handelt sich um ein erfindungsgemäßes Konzept eines dynamischen Legierens. Dabei wird eine Adaption der Elementgrenzen für Legierungselemente in der Flüssigphase durchgeführt.

Durch eine Analyse der Schmelze werden Ist-Werte der Elementgehalte bestimmt. Diese Ist-Werte werden in ein für eine spezifizierte Stabstahlgüte passendes Prognosemodell eingespeist, sodass der Einfluss der jeweiligen Elemente auf den Härteverlauf unter Anwendung einer multiplen linearen Regression modelliert und, wenn dies zur Sicherstellung eines gewünschten Härteverlaufbereichs notwendig ist, durch ein Korrekturmodell eine Reaktion in Form von adaptierten Legierungselementgrenzen aufgefunden wird und diese dem Legierungsrechner als adaptierte Zielwerte zugeführt werden. Der Legierungsrechner benutzt die adaptierten Legierungselementgrenzen zur Festlegung der Massen an Einsatzstoffen.

Die Erfinder haben erkannt, dass bei der dynamischen Korrektur der Elementgehalte zwischen zwei definierten Elementarten unterschieden werden soll. Es handelt sich hierbei um Einfluss- und Kompensationselemente.

Bei der Stahlherstellung werden beim Herstellungsprozess verschiedene Einsatzstoffe verwendet. Dies sind zum Beispiel Eisenerz, Eisenschwamm in Form von direkt reduziertem Eisen (DRI) bzw. heiß brikettiertem Eisen (HBI) und Schrott. Über diese Einsatzstoffe wird natürlich nicht nur Eisen eingebracht, sondern auch sogenannte Begleitelemente und Spurenelemente.

Begleitelemente sind üblicherweise nicht vermeidbare Elemente, wie beispielsweise Si, Mn, S, P, O, N und H, und werden im Verlauf eines nachfolgenden metallurgischen Prozesses entfernt, sofern das möglich ist. Ist ein Entfernen nicht möglich, müssen das jeweilige Begleitelement und sein Einfluss bei metallurgischen Prozessen berücksichtigt werden.

Für jede Stahlsorte, welche durch die chemische Zusammensetzung der Legierung und die Herstellroute definiert wird, existiert eine definierte, aus praktischen Erfahrungswerten resultierende Begleitelementklasse, d.h. es müssen bestimmte Obergrenzen der Gehalte der Begleitelemente eingehalten werden.

Bei Spurenelementen handelt es sich um explizit störende Begleitelemente (z.B. As und Sb), welche in sehr geringen Mengen vorliegen. Diese werden im Zuge des (hier durchgeführten dynamischen) Legierens nicht berücksichtigt.

Legierungselemente sind Elemente, welche gezielt zugesetzt werden, um dem Stahl gewünschte, werkstoffkundliche Eigenschaften zu verleihen. Solche Eigenschaften können mechanische Eigenschaften wie Härte, Zugfestigkeit, Zähigkeit und weitere Eigenschaften sein, aber auch chemische Eigenschaften wie Widerstand gegen Korrosion oder Wasserstoffversprödung und weitere.

Bei Einsatz von Einsatzstoffen, insbesondere metallischen Einsatzstoffen und nicht metallischen Einsatzstoffen, muss beachtet werden, dass sie für sich gesehen Begleitelemente aus ihrer Rohstoffhistorie und Legierungselemente aus ihrer metallurgischen Historie enthalten.

Wird nun beispielsweise Schrott als Einsatzstoff in der Stahlherstellung verwendet, sind seine Bestandteile, bis auf Eisen, für das Produkt, was unter Einsatz des Schrotts erzeugt werden soll, Begleit- und Spurenelemente.

Deren Anwesenheit muss also berücksichtigt werden, da sie (als ehemalige Legierungselemente) aus dem Produkt über metallurgische Prozesse entweder nur mit großem Aufwand oder nicht entfernbar sind. Jedoch kann ihre Wirkung erfindungsgemäß berücksichtigt und kompensiert werden.

Im klassischen Herstellungsprozess wird von einem Produkt (Stahl) ausgegangen, welches entsprechend den Marktanforderungen bestimmte werkstoffkundliche Eigenschaften aufweist. Auf dieses Anforderungsprofil hin, wird es chemisch/metallurgisch eingestellt. Ein solches Produkt ist also eine Legierung, wobei die Legierungsbestandteile durch einen definierten Gehalt an Obergrenzen oder mit Unter- und Obergrenzen bestimmt sind. Von einer bestimmten Legierung ist somit bekannt, welche Eigenschaften sie haben kann.

Wird nun aufgrund geänderter Bedingungen, der Einsatz von Einsatzstoffen wie bspw. Schrott erheblich erhöht, wird das Produkt folgerichtig mit einer höheren Menge von Begleitelementen befrachtet. Diese Begleitelemente haben (als "ehemalige" Legierungselemente) einen Einfluss zum Beispiel auf werkstoffkundliche Eigenschaften des Produkts.

Insofern muss dies bei dem Zulegieren berücksichtigt werden. Hierbei können die folgenden Fälle eintreten.
A) Beispielsweise wird für Element A durch den Einsatzstoff ein Anteil eingebracht, der unterhalb des gewünschten Gehalts liegt. In diesem Fall muss ggf. weniger Element A zulegiert werden.
B) Für Element A wird über den Einsatzstoff ein Gehalt eingebracht, der bereits im Zielfenster liegt, es muss nichts mehr zulegiert werden.
C) Es wird mehr Element A als gewünscht eingebracht. Der Gehalt des Elements A kann nur unter erhöhtem Aufwand metallurgisch verringert werden. Dementsprechend muss die Wirkung des Elements A kompensiert werden.

Das obige Beispiel wird dann komplexer, wenn mehrere Begleitelemente oder Legierungselemente für bestimmte Eigenschaften zusammenspielen.

Hier setzt die Erfindung dadurch an, dass zu den Begleitelementen, die einen Einfluss auf den Härteverlauf haben (nachfolgend Einflusselemente genannt), Legierungselemente ausgewählt werden, die den Einfluss des Einflusselements auf den Härteverlauf kompensieren können (nachfolgend Kompensationselemente genannt).

Kompensationselemente sind Elemente, die bestenfalls ohnehin reguläre Legierungselemente der Ziellegierung sind, sodass im Rahmen der qualitativen gewünschten Legierungszusammensetzung lediglich quantitative Anpassungen erfolgen.

Ein einfaches Beispiel soll dies deutlich machen. Sind neben Eisen in einer gewünschten Stahllegierung die Elemente A, B und C enthalten und ist das Element A in einer Menge vorhanden, die die gewünschte Menge übersteigt, und ist C ein Element, welches den Einfluss von A auf den Härteverlauf verringert, wird C in einer Menge zulegiert, welche zusätzlich zur eigenen Wirkung noch die Wirkung von A kompensiert.

Ein weiteres Beispiel ist, wenn Element A durch die Einsatzstoffe in einer Menge vorhanden ist, die die gewünschte Menge überschreitet. Wenn das Element C einen vergleichbaren Einfluss auf den Härteverlauf hat wie A, jedoch in der Legierung wenig oder nur in Spuren vorhanden ist, wird hiervon nur so viel zugesetzt, dass die addierte Wirkung von A und C gemeinsam den durch die erwünschte Stabstahlgüte spezifizierten Härteverlauf zur Folge haben. D.h. in diesem Fall, dass weniger C zugesetzt wird als normalerweise zugesetzt würde, um den zu hohen A-Gehalt zu kompensieren.

Kompensation im Sinne der Erfindung bedeutet somit, dass eine Anpassung der Ziellegierungszusammensetzung nach oben oder unten stattfinden kann.

B ist in diesem Fall z.B. ein Element, welches auf den Härteverlauf keinen Einfluss hat.

Insgesamt kann durch dieses erfindungsgemäße Vorgehen, die Einsatzstoffvorauswahl gröber erfolgen.

Eingangskenngrößen in das Prognosemodell sind alle Elemente, die in der Flüssigphase enthalten und analysiert worden sind.

Ausgangsgrößen sind adaptierte Elementgrenzen für Elemente, welche in der Flüssigphase legiert werden. Es handelt sich hierbei um die sogenannten Kompensationselemente. Kompensationselemente sind somit Elemente, welche in der Flüssigphase zulegiert werden, um den Einfluss der Einflusselemente auszugleichen, damit die Soll-Eigenschaften der Endlegierung erreicht werden.

Sowohl die Einfluss- als auch die Kompensationselemente sind Elemente, welche durch Zugabe von Einsatzstoffen in die Schmelze eingebracht werden.

Dabei handelt es sich um Begleitelemente, die im Eisen oder Stahl unvermeidlich vorkommen und in geringer Menge enthalten sein können. Sie können aus den verwendeten Rohstoffen, wie Erz oder Schrott, stammen oder bei der Verarbeitung in die Schmelze eingebracht werden. Diese Begleitelemente beeinflussen die Eigenschaften des Stahls und können je nach Gehalt positive oder negative Effekte auf den Härteverlauf haben.

In der Stahlherstellung wird "Schrott" als recycelter, wiederverwendbarer metallischer Sekundärstoff bezeichnet, der als Einsatzstoff zur Produktion von neuem Stahl dient. Schrott besteht hauptsächlich aus Eisenbasislegierung und wird in verschiedenen Qualitäten und Sorten klassifiziert, je nach Reinheit, Form, Größe und chemischer Zusammensetzung.

Das vorliegende Verfahren, bei dem Einsatzstoffe eingesetzt werden, betrifft die Herstellung von Stabstählen mit einem auf eine Stabstahlgüte spezifizierten Härteverlauf.

Das Konzept beruht darauf, ein Endprodukt einer bestimmten Stabstahlgüte zu erzeugen, bei denen sich der Härteverlauf trotzt erhöhter Einflusselementgehalte, welche durch die Einsatzstoffen in metallurgischen Aggregaten eingebracht werden, gar nicht oder nur möglichst geringfügig ändert und zwar im Vergleich zu dem Härteverlauf, der sich einstellt, wenn bei der Herstellung der Schmelzen Einsatzstoffe mit geringen Anteilen an Einflusselementen eingesetzt werden und damit geringere Anteile an unerwünschten Begleitelementen eingebracht werden (z.B. Primärroute), so dass das Produkt innerhalb eines durch die Stabstahlgüte spezifizierten Härteverlaufbereichs liegt. Dies stellt auch sicher, dass auf die Stahlherstellung folgende Nachbehandlungsschritte nicht geändert werden müssen.

Dies geschieht durch Adaption eines oder mehrerer Legierungselemente, und wird nachfolgend als dynamisches Legieren bezeichnet. Die Adaption erfolgt durch Anwendung eines Prognosemodells, welches zunächst aufgrund von Analyse-Ist-Werten den erwartbaren Härteverlauf modelliert. Sollte dieser außerhalb des durch die gewünschte Stabstahlgüte spezifizierten Härteverlaufbereichs liegen, werden mittels eines Korrekturmodells korrigierte Legierungselementgehalte berechnet, sodass die spezifizierten Anforderungen an den Härteverlauf erfüllt werden können.

Der Härteverlauf (vgl. Bild 5 der EN ISO 642) ergibt sich aus positionsabhängigen Härtewerten HRCₙ, welche an bestimmten Positionen n (Abstand von der Oberfläche in mm) mithilfe der folgenden Formel ermittelt werden können: ${HRC}_{n} = {K}_{n} + {\sum }_{i = 1}^{m} {k}_{i , n} ∗ Gew . - {%}_{i}$ wobei
- n: der Abstand des Härtewertes von der Oberfläche in mm bis in eine Tiefe von 50 mm,
- Kₙ: der Grundhärtewert im Abstand n von der Oberfläche,
- k_{i,n}: der Gewichtungsfaktor, der den Einfluss eines bestimmten Elements i auf die Härte im Abstand n von der Oberfläche wiedergibt,
- m: die Anzahl der berücksichtigten Elemente, und
- Gew.-%ᵢ: der Gehalt des jeweiligen Elements i ist.

Für die Erzeugung zumindest eines Prognosemodells und/oder Korrekturmodells müssen Trainingsdaten zur Verfügung stehen. Diese beruhen beispielsweise auf realen Messwerten aus Stirnabschreckversuchen (Jominy-Versuchen; Bestimmung nach EN ISO 642).

Zunächst wird eine Silhouetten-Analyse zur Bestimmung der optimalen Clusteranzahl bzw. Anzahl an Prognosemodellen und/oder Korrekturmodellen durchgeführt. Die Cluster definieren dabei eine Gruppe von Datenpunkten, welche als Datenbasis für die multiple lineare Regression herangezogen werden.

Es zeigt sich dabei, dass eine sechsteilige Clusterung, d.h. sechs Prognosemodelle und/oder Korrekturmodelle, unter Berücksichtigung der Elemente (C, Si, Mn, P, S, Cu, Cr, Ni, Mo, V, Ti, Al, B, Nb, W, N, Co, Pb) und insbesondere deren Gehalte in Gew.-% optimal ist. Zusätzlich zu den sechs automatisch generierten Clustergruppen werden drei Prognosemodelle und/oder Korrekturmodelle basierend auf durch die Ziellegierung definierten Filtern empirisch bzw. manuell erzeugt:
- Modell 1 "unlegiert": Eignung für Stähle mit geringen Kohlenstoff- und Chromgehalten, insbesondere für Analysen mit C ≤ 0,2 Gew.-%, Cr ≤ 1,3 Gew.-%, B ≤ 0,0008 Gew.-% und Mn ≤ 1,5 Gew.-%;
- Modell 2 "legiert": Eignung für Stähle mit höheren Kohlenstoff-, Chrom- und Mangangehalten, insbesondere für Analysen mit C ≤ 0,9 Gew.-%, 0,8 Gew.-% ≤ Cr ≤ 2,3 Gew.-%, B ≤ 0,0008 Gew.-% und Mn ≤ 1,5 Gew.-%;
- Modell 3 "Bor-legiert": Eignung für Stähle mit erhöhtem Borgehalten, insbesondere für Analysen mit B > 0,0008 Gew.-%, Si ≤ 0,5 Gew.-% und Mn ≤ 1,7 Gew.-%, wobei zusätzlich ein Borpotential B_{P} berücksichtigt wird.

Das Borpotential B_{P} ergibt sich dabei gemäß folgender Formel: ${B}_{p} = Gew . - {%}_{B} - \frac{{M}_{B}}{{M}_{N}} \times \left(Gew.-{%}_{N}-\frac{{M}_{N}}{{M}_{Ti}}\times Gew.-{%}_{Ti}\right) ,$ wobei
- Gew.-%ᵢ: der Gehalt des jeweiligen Elements i und
- Mᵢ: die molare Masse des jeweiligen Elements i in g/mol ist.

Weitere Prognosemodelle und/oder Korrekturmodelle werden mittels maschinellen Lernens, insbesondere unüberwachten maschinellen Lernens erzeugt. Unüberwachtes Lernen (unsupervised learning) bezeichnet maschinelles Lernen üblicherweise ohne im Voraus bekannte Zielwerte sowie ohne "Belohnung" durch die Umwelt. Eine der Hauptaufgaben des unüberwachten Lernens ist die Identifikation von Clustern in unstrukturierten Daten. Hierbei existieren verschiedene Vorgehensweisen, wie die Einteilung in diese Cluster geschehen kann. Die Clusteranalyse unterscheidet hierbei unter anderem zwischen hierarchischem (Minimum Linkage, Maximum Linkage, Ward Linkage), partitionierendem (K-Means, k-Medoids, Fuzzy-c-means, CLARA, Mini-Batch K-Means, Bisecting K-Means, EM-Clustering), dichtebasierendem (DBSCAN) und gitterbasierendem (STING, CLIQUE) Clustering.

Die Bestimmung der optimalen Anzahl von Clustern in einem Datensatz dient zur Vermeidung von Über- oder Untersegmentierung. Um diese optimale Clusterzahl zu bestimmen, können folgende Verfahren und Ansätze genutzt werden: Elbow-Method (Ellenbogenmethode), Silhouetten-Analyse, Entropiemethode, Intra-Cluster-Varianz (WCSS), Gap-Statistik, Davies-Bouldin-Index, Calinski-Harabasz-Index (Variation Ratio Criterion), informationstheoretische Kriterien (Akaike-Informationskriterium oder Bayesianische Informationskriterium), Hierarchische Clusteranalyse mit Dendrogramm, Modelbasiertes Clustering wie Gaussian Mixture Models (GMM).

Um ein adäquates Ergebnis der Clusteranalyse zu erhalten, sind fehlende oder fehlerhafte Werte in den Datensätzen zu bereinigen. Bei stark unterschiedlichen Wertebereichen ist eine vorherige z-Transformation der Variablen durchzuführen.

Die Wahl des Proximitätsmaßes (Ähnlichkeits- oder Distanzmaß) entscheidet über die Qualität der Clusteranalyse und wird daher an die Art der Daten (metrisch, ordinal, nominal) angepasst.

Für metrische (numerische) Daten werden die euklidische Distanz, die quadrierte euklidische Distanz, die L1-Distanz (auch Manhattan-Metrik, Manhattan-Distanz, Mannheimer Metrik, Taxi- oder Cityblock-Metrik), die Minkowski-Distanz, die Manhalanobis-Distanz oder die Cosinus-Ähnlichkeit verwendet.

Für nominale (kategorische) Daten werden die Hamming-Distanz, der Jaccard-Index, der Matching-Koeffizient oder der Gower-Koeffizient genutzt.

Für ordinale Daten (natürliche Reihenfolge, aber ohne gleichen Abstand zwischen Kategorien) werden der Spearman-Koeffizient oder die Ordinal Gower-Metrik angewendet.

Bei gemischten Daten kann der Gower-Koeffizient, der Distance-Weighted Gower-Koeffizient, Daisy, die Kombination von Distanzmaßen, heterogene Distanzfunktionen, die Feature Transformation, Kohonen's Self-Organizing Maps (SOMs) oder ein probabilistischer Ansatz (Gaussian Mixture Models oder Latent Class Analysis) verwendet werden.

Die sechs weiteren Prognosemodelle und/oder Korrekturmodelle werden mittels der Silhouettenanalyse und der sogenannten "K-Means"-Methode automatisch erzeugt.

Die "K-Means"-Methode ist ein zentroidbasierter Clustering-Algorithmus, bei dem der Abstand zwischen jedem Datenpunkt und einem Zentroid (Clusterzentrum oder Clusterschwerpunkt) berechnet (minimiert) wird, um ihn einem Cluster zuzuordnen. Ziel ist es, n Beobachtungen (Datenpunkte) in K Cluster aufzuteilen und demzufolge, die K-Anzahl von Cluster im Datensatz zu ermitteln. Es handelt sich um einen iterativen Prozess, bei dem jeder Datenpunkt den Gruppen zugewiesen wird und die Datenpunkte nach und nach anhand ähnlicher Merkmale (z.B. Gew.-% eines Legierungselements) geclustert werden. Dabei soll die Summe der Abstände zwischen den Datenpunkten und dem Clusterschwerpunkt minimiert werden, um die richtige Gruppe zu ermitteln, zu der jeder Datenpunkt gehört.

Der Algorithmus kann auf fünf Schritte heruntergebrochen werden:
1) Wählen der Clusteranzahl K, in denen die Datenpunkte n gruppiert werden;
2) Initialisierung der Clusterschwerpunkte C: Der Schwerpunkt ist das Zentrum eines Clusters, aber zu Beginn des Verfahrens ist das genaue Zentrum der Datenpunkte unbekannt. Insofern wählt man zufällige Datenpunkte (gleiche Anzahl wie Cluster) aus und definiert sie als Schwerpunkte für die jeweiligen Cluster;
3) Datenpunkte dem nächstgelegenen Cluster zuordnen: Nach der Initialisierung der Clusterschwerpunkte, besteht der nächste Schritt in der Zuordnung der Datenpunkte zu den nächstgelegenen Clusterschwerpunkten. Zunächst wird der Abstand zwischen dem Datenpunkt und dem Schwerpunkt unter Verwendung der euklidischen Distanzmetrik berechnet. Dann wird der Cluster für die Datenpunkte ausgewählt, bei dem der Abstand zwischen dem Datenpunkt und dem Schwerpunkt ein Minimum aufweist;
4) Neuinitialisierung der Zentren: Die Zentren werden neu initialisiert, indem der Durchschnitt aller Datenpunkte des jeweiligen Clusters berechnet wird;
5) Wiederholen der Schritte 3 und 4, bis die optimalen Clusterschwerpunkte vorliegen und sich die Zuordnungen der Datenpunkte zu den richtigen Clustern nicht mehr ändern.

Die den Clustern zugeordneten Daten werden dann als Datenbasis für die multiple lineare Regression verwendet.

Jeder durch Analyse der Schmelze bestimmte Ist-Wert wird nun in jedes der Prognosemodelle eingespeist. Das Prognosemodell und/oder Korrekturmodell mit der höchsten Genauigkeit, welches sich durch die geringste Abweichung/den geringsten Wert des Mittelwerts und/oder der Summe der Quadratwurzeln der mittleren quadratischen Fehler (root mean square error, RMSE) der Härtewerte je Abstand n auszeichnet, wird schließlich zur Modellierung des Härteverlaufs unter Verwendung obiger Formel zur Berechnung der prädiktiven Härtewerte HRCₙ herangezogen.

Die Modellierung des Härteverlaufs zeigt, ob dynamisches Legieren notwendig ist, damit der Härteverlauf in einen spezifizierten Härteverlaufbereich fällt. Liegt der Härteverlauf im vorgesehenen Bereich, findet keine Kompensation der Einflusselemente durch ausgewählte Kompensationselemente statt. Weicht der Härteverlauf jedoch vom vorgesehenen Härteverlaufbereich ab, werden durch das Korrekturmodell adaptierte Elementgehalte der Ziellegierung berechnet. Entsprechend wird das zumindest eine Kompensationselement zulegiert, um den Härteverlauf in den Zielbereich zu bringen.

Nach der Zugabe von Kompensationselementen kann eine weitere Analyse getätigt werden. Der Ist-Wert der Legierungszusammensetzung wird kontrolliert und bei Bedarf erneut dynamisch mithilfe des Korrekturmodells korrigiert.

Die Elemente Cu, Mo, Sn und Ni werden als Einflusselemente betrachtet. Die übrigen Elemente werden als Kompensationselemente betrachtet.

Hiervon ausgenommen sind zum Beispiel bestimmte legierte Stähle wie folgt: Bei Nickellegierten Stabstahlgüten zählen nur Cu, Mo, Sn, bei Mo-legierten Stabstahlgüten nur Cu, Sn, Ni und bei Ni-Mo-legierten Stabstahlgüten nur Cu, Sn zu den Einflusselementen. In diesen Fällen sind entsprechend die zuvor genannten Legierungselemente auch als Kompensationselemente geeignet.

Der Einfluss der einzelnen Elemente auf die werkstoffkundlichen Eigenschaften der Legierung ist wie folgt.

Kupfer wird als Legierungselement in herkömmlichen Stählen aufgrund der Gefahr von Duktilitätsverlust und interkristallinen Oberflächenrissen nur in äußerst geringen Mengen zugesetzt. Kupfer hat zudem einen negativen Einfluss auf die Kaltverformbarkeit, Warmumformbarkeit und Kerbschlagzähigkeit und vermindert die Schweißneigung. Zu hohe Kupfergehalte können zu unerwünschter Rotbrüchigkeit führen, welche bei der Warmumformung durch Bildung einer Kupferschmelze aufgrund von selektiver Korrosion auftritt und bei geringsten Belastungen zu Rissen oder Brüchen führen kann. Der Rotbrüchigkeit kann z.B. durch Zulegieren von Nickel entgegengewirkt werden. Andererseits kann Kupfer die Streckgrenze, Zugfestigkeit und Härtbarkeit steigern. Durch Ausscheidungshärtung trägt Kupfer effektiv zur Festigkeitssteigerung bei. Bei wetterfesten Stählen kann ein Zulegieren von Kupfer die Witterungs- und Korrosionsbeständigkeit erhöhen.

Es ist vorteilhaft, wenn ein Kupfergehalt von 0,001 bis 0,4 Gew.-%, vorzugsweise 0,001 bis 0,38 Gew.-%, besonders bevorzugt 0,001 bis 0,35 Gew.-%, weiterhin besonders bevorzugt 0,001 bis 0,3 Gew.-% gewählt wird.

Besonders über die EAF-Route erzeugte Stahlschmelzen weisen aufgrund des erhöhten Schrotteinsatzes oft zu hohe Kupfergehalte auf. Die Entfernung von Kupfer aus der Schmelze ist äußerst schwierig, sodass seine Wirkung durch andere Legierungselemente kompensiert werden muss.

Nickel erhöht durch die Verringerung der kritischen Abkühlgeschwindigkeit die Durchhärtung und Durchvergütung. Zudem erhöht es die Zähigkeit sowie die Härte und Festigkeit von Stahl. Darüber hinaus erhöht Nickel die Löslichkeit von Kupfer im Stahl, sodass unerwünschter Rotbrüchigkeit entgegengewirkt wird. Nachteilig sind jedoch die hohen Kosten, die bei Verwendung von Nickel als Legierungselement entstehen.

Es ist vorteilhaft, wenn ein Nickelgehalt von 0,001 bis 2,5 Gew.-%, vorzugsweise 0,001 bis 2,0 Gew.-%, bevorzugt 0,001 bis 1,5 Gew.-%, besonders bevorzugt 0,001 bis 1,0 Gew.-% gewählt wird.

Molybdän steigert die Festigkeit und Härtbarkeit des Stahls, während es jedoch die Duktilität aufgrund seiner Neigung zu Karbidbildung verringert, wodurch die Warmfestigkeit verbessert wird. Seine festigkeitssteigernde Wirkung hat direkten Einfluss auf den Härteverlauf. Molybdänkarbide erhöhen außerdem die Verschleißfestigkeit und Anlassbeständigkeit. Auch die Lochkorrosions- und Zunderbeständigkeit von Stahl kann durch Molybdän verbessert werden. Molybdän ist ein Ferritstabilisator und begünstigt dadurch die Phasenumwandlung von Austenit zu Ferrit, wodurch sich der Ferritbereich im Phasendiagramm vergrößert. Darüber hinaus wird die Schweißbarkeit des Stahles mit steigenden Molybdängehalt vermindert.

Es ist vorteilhaft, wenn ein Molybdängehalt von 0,001 bis 1,0 Gew.-%, vorzugsweise 0,001 bis 0,8 Gew.-%, besonders bevorzugt 0,001 bis 0,75 Gew.-% gewählt wird.

Zinn wirkt verfestigend und härtesteigernd, vermindert zugleich die Bruchdehnung und neigt zu stärkeren Seigerungen, wodurch die Gefügestabilität beeinflusst wird. Zinn wirkt darüber hinaus oberflächenaktiv und verschlechtert die Warmumformbarkeit.

Es ist vorteilhaft, wenn ein Zinngehalt von 0,001 bis 0,03 Gew.-%, vorzugsweise 0,001 bis 0,028 Gew.-%, bevorzugt 0,001 bis 0,026 Gew.-%, besonders bevorzugt 0,001 bis 0,024 Gew.-% gewählt wird.

Kohlenstoff bildet Karbide mit den unterschiedlichsten Elementen (z.B. Zementit, Fe₃C). Karbidbildung erhöht die Härte. Daher ist die Härte direkt proportional zum Kohlenstoffgehalt. Geringe Kohlenstoffgehalte sichern eine gute Umformbarkeit und eine gute Schweißbarkeit, können jedoch keine hohe Festigkeit bedingen. Hohe Kohlenstoffgehalte erhöhen die Festigkeit, was aber auch die Gefahr der Versprödung birgt. Eine der wichtigsten Eigenschaften des Kohlenstoffs als Legierungselement ist die Möglichkeit, umwandlungsfähigen Stahl durch Abschreckung zu härten.

Es ist vorteilhaft, wenn ein Kohlenstoffgehalt von 0,05 bis 1,3 Gew.-%, vorzugsweise 0,05 bis 1,30 Gew.-%, bevorzugt 0,05 bis 1,25 Gew.-%, besonders bevorzugt 0,05 bis 1,22 Gew.-%, weiterhin besonders bevorzugt 0,05 bis 1,20 Gew.-% gewählt wird.

Silizium hat einen günstigen Einfluss auf die Duktilität und die Festigkeit des Stahls. Zudem verbessert es die Zunderbeständigkeit und vermindert so die Gefahr der Rotbrüchigkeit. Aufgrund seiner hohen Sauerstoffaffinität ist es ein wichtiges Desoxidationsmittel. Hohe Siliziumgehalte führen durch Mischkristallbildung zur Verfestigung des Gefüges. Bei zu hohen Gehalten kann es jedoch eine schädigende Wirkung bspw. auf die Kaltumformbarkeit haben. Silizium erhöht die Streckgrenze und die Zugfestigkeit, ohne die Dehnung wesentlich zu verringern. Weiter vermindert das Element die kritische Abkühlgeschwindigkeit und vergrößert damit die Einhärtung. Silizium erhöht auch die Zunderbeständigkeit und hat einen signifikanten Einfluss auf die elektrischen Eigenschaften des Stahls.

Es ist vorteilhaft, wenn ein Siliziumgehalt von 0,05 bis 1,2 Gew.-%, vorzugsweise 0,05 bis 1,15 Gew.-%, bevorzugt 0,05 bis 1,10 Gew.-%, besonders bevorzugt 0,05 bis 1,05 Gew.-%, weiterhin besonders bevorzugt 0,05 bis 1,0 Gew.-% gewählt wird.

Mangan hat bei niedrigen Gehalten eine vorteilhafte Wirkung auf das Seigerungsverhalten im Strangguss und verbessert die Umformbarkeit. Bezüglich Härtbarkeit und Durchhärtung ist Mangan eines der billigsten und wirkungsvollsten Legierungselemente. Mit der Herabsetzung der kritischen Abkühlgeschwindigkeit bei zunehmendem Mangangehalt ist eine Erhöhung der Einhärtung verbunden. Höhere Mangangehalte bedingen eine höhere Grundfestigkeit, erhöhen aber auch das Risiko der Wasserstoffversprödung. Mangan ist ein wirksames Desoxidationsmittel und ein starker Austenitstabilisator. Es ist zur Vermeidung von Rotbrüchigkeit üblicherweise in allen warmgewalzten Stählen enthalten.

Es ist vorteilhaft, wenn ein Mangangehalt von 0,1 bis 2,0 Gew.-%, vorzugsweise 0,1 bis 1,9 Gew.-%, bevorzugt 0,1 bis 1,8 Gew.-%, besonders bevorzugt 0,1 bis 1,7 Gew.-%, weiterhin besonders bevorzugt 0,1 bis 1,6 Gew.-% gewählt wird.

Chrom erhöht die Zugfestigkeit, während die Dehnung nur geringfügig verschlechtert wird. Durch Herabsetzen der kritischen Abkühlgeschwindigkeit wird die Einhärtbarkeit wesentlich gesteigert. Zusätzlich wird die Härte durch Karbidbildung erhöht. Höhere Chromgehalte verbessern auch die Warmfestigkeit und Anlassbeständigkeit. Chrom hat zudem eine positive Wirkung auf die Korrosionsbeständigkeit. Darüber hinaus hat Chrom einen negativen Einfluss auf die Schweißbarkeit.

Es ist vorteilhaft, wenn ein Chromgehalt von 0,001 bis 2,0 Gew.-%, vorzugsweise 0,001 bis 1,9 Gew.-%, bevorzugt 0,001 bis 1,8 Gew.-%, besonders bevorzugt 0,001 bis 1,7 Gew.-%, weiterhin besonders bevorzugt 0,001 bis 1,6 Gew.-% gewählt wird.

Vanadium hat eine härtende Wirkung und ermöglicht so das Bainitisieren bei höheren Temperaturen. Zu hohe Vanadiumgehalte führen zu Ausscheidungen, welche die Widerstandsfähigkeit des Stahls gegen verzögerten Wasserstoffbruch beeinträchtigen.

Es ist vorteilhaft, wenn ein Vanadiumgehalt von 0,001 bis 0,4 Gew.-%, vorzugsweise 0,001 bis 0,40 Gew.-%, bevorzugt 0,001 bis 0,38 Gew.-%, besonders bevorzugt 0,001 bis 0,36 Gew.-%, weiterhin besonders bevorzugt 0,001 bis 0,34 Gew.-% gewählt wird.

Aluminium ist ein wichtiges Desoxidationsmittel, insbesondere in Stählen mit niedrigen Mangan- und Siliziumgehalten. Durch die Bildung von Aluminiumnitriden kann die austenitische Kornvergröberung beim Warmwalzen kontrolliert werden. Zu hohe Aluminiumgehalten führen allerdings zu einer Vergröberung von aluminatartigen Einschlüssen im Stahl, die insbesondere für die Zähigkeit schädlich sein können.

Es ist vorteilhaft, wenn ein Aluminiumgehalt von 0,0005 bis 0,05 Gew.-%, vorzugsweise 0,0005 bis 0,04 Gew.-%, bevorzugt 0,0005 bis 0,03 Gew.-%, besonders bevorzugt 0,0005 bis 0,02 Gew.-%, weiterhin besonders bevorzugt 0,0005 bis 0,01 Gew.-% gewählt wird.

Bor verbessert die Härtbarkeit und senkt bei Cu-legierten Stählen die Gefahr der Rotbrüchigkeit. Durch Seigerungen an den früheren austenitischen Korngrenzen erhöht Bor zudem die Widerstandsfähigkeit gegen Wasserstoffbruch. Bor wirkt in Synergie mit Molybdän und Niob und erhöht so die Wirksamkeit dieser Elemente. Höhere Borgehalte sind jedoch zu vermeiden, da diese zur Bildung von spröden Eisenborkarbiden führen.

Es ist vorteilhaft, wenn ein Borgehalt von 0,0005 bis 0,008 Gew.-%, vorzugsweise 0,0005 bis 0,007 Gew.-%, besonders bevorzugt 0,0005 bis 0,006 Gew.-% gewählt wird.

Titan kann unerwünschten Stickstoff abbinden und trägt zur Ausscheidungshärtung bei. Es erhöht auch die verzögerte Bruchfestigkeit. Nachteilig verbunden mit höheren Ti-Gehalten ist die Abbindung des gelösten Bors in Form von Titanboriden, die sich bereits bei hohen Temperaturen bilden.

Es ist vorteilhaft, wenn ein Titangehalt von 0,001 bis 0,04 Gew.-%, vorzugsweise 0,001 bis 0,03 Gew.-%, bevorzugt 0,001 bis 0,02 Gew.-%, besonders bevorzugt 0,001 bis 0,01 Gew.-%, weiterhin besonders bevorzugt 0,001 bis 0,005 Gew.-% gewählt wird.

Niob erhöht die Wasserstoffbeständigkeit und unterstützt eine gute Verteilung schädlicher Elemente wie Phosphor und Schwefel im Stahl. Hohe Niobgehalte führen jedoch zu großen Ausscheidungen, die die Beständigkeit des Stahls gegen Spätbruch beeinträchtigen. Außerdem führt ein zu hoher Niobgehalt zu einem erhöhten Risiko von "Riss"-Fehlern an der Oberfläche der Knüppel und Vorblöcke beim Stranggießen.

Es ist vorteilhaft, wenn ein Niobgehalt von 0,001 bis 0,1 Gew.-%, vorzugsweise 0,001 bis 0,09 Gew.-%, bevorzugt 0,001 bis 0,08 Gew.-%, besonders bevorzugt 0,001 bis 0,07 Gew.-%, weiterhin besonders bevorzugt 0,001 bis 0,06 Gew.-% gewählt wird.

In geringen Mengen kann Stickstoff dazu beitragen, eine übermäßige Vergröberung des Austenitkorns während der Wärmebehandlung des Stahls zu vermeiden. Zudem ermöglicht die Bildung von Karbonitriden den Einschluss von Wasserstoff. Als Austenitbildner kann Stickstoff Nickel und Kohlenstoff teilweise substituieren. In Bor-legierten Stählen bindet Stickstoff Bor durch die Bildung von Bornitriden, sodass die positive Wirkung von Bor auf die Härtbarkeit des Stahls verloren geht. Daher ist der Stickstoffgehalt im borlegierten Stählen auf 0,01 Gew.-% begrenzt.

Es ist vorteilhaft, wenn ein Stickstoffgehalt von 0,0001 bis 0,02 Gew.-%, vorzugsweise 0,0001 bis 0,015 Gew.-%, bevorzugt 0,0001 bis 0,01 Gew.-%, besonders bevorzugt 0,0001 bis 0,005 Gew.-%, weiterhin besonders bevorzugt 0,0001 bis 0,001 Gew.-% gewählt wird.

Wolfram ist ein starker Karbidbildner und erhöht so die Härte des Stahls. Es erhöht zudem sie Warmfestigkeit, Anlassbeständigkeit und die Verschleißfestigkeit bei hohen Temperaturen.

Es ist vorteilhaft, wenn ein Wolframgehalt von 0,001 bis 0,5 Gew.-%, vorzugsweise 0,001 bis 0,4 Gew.-%, bevorzugt 0,001 bis 0,3 Gew.-%, besonders bevorzugt 0,001 bis 0,2 Gew.-%, weiterhin besonders bevorzugt 0,001 bis 0,1 Gew.-% gewählt wird.

Kobalt ist ein Austenitbildner, es hemmt das Kornwachstum bei hohen Temperaturen und erhöht stark die Warmfestigkeit durch verbesserte Anlassbeständigkeit.

Es ist es vorteilhaft, wenn ein Kobaltgehalt von 0,001 bis 0,2 Gew.-%, vorzugsweise 0,001 bis 0,15 Gew.-%, besonders bevorzugt 0,001 bis 0,1 Gew.-% gewählt wird.

Da Blei im Eisen völlig unlösbar ist, verteilt es sich bei Zugabe dispersionsförmig im Stahl und liegt als einzelne Phase dort vor. Diese feinverteilten Bleieinschlüsse verbessern die Spanbarkeit in Automatenstahl.

Es ist vorteilhaft, wenn ein Bleigehalt von 0,001 bis 0,4 Gew.-% vorzugsweise 0,001 bis 0,3 Gew.-%, bevorzugt 0,001 bis 0,2 Gew.-%, besonders bevorzugt 0,001 bis 0,1 Gew.-%, weiterhin besonders bevorzugt 0,001 bis 0,05 Gew.-% gewählt wird.

Die Wirkung von Phosphor und Schwefel ist in Stabstählen besonders ungünstig, da diese Elemente zu Seigerungen an den Korngrenzen führen, welche die Gefahr von Korngrenzenrissen erhöhen. Zudem erhöhen Phosphor und Schwefel das Risiko für Heißrisse. Die Gehalte von Phosphor und Schwefel müssen daher sehr niedrig gehalten werden.

Es ist zu beachten, dass die schädliche Wirkung von Einflusselementen vor allem in Kombination untereinander als kritisch zu sehen ist, wobei deren Ursprung in entsprechenden Einsatzmaterialverunreinigung (beim Einsatzmix zur Erstellung der Schmelze) begründet ist. Bei Verwendung von Schmelztechnologien mit erhöhten Mengen an Sekundärrohstoffen können somit durch die unweigerlich erhöhte Einflusselementfracht größere Schwankungsbreiten bezüglich des Härteverlaufs auftreten.

Die Erfindung betrifft somit ein Verfahren zur Herstellung von Stabstählen mit einem auf eine Stabstahlgüte spezifizierten Härteverlaufbereich, welcher durch eine Ziellegierung sichergestellt wird, wobei zumindest ein primärmetallurgisches Aggregat zu einer ersten Schmelze führt, wobei aus der ersten Schmelze zumindest eine Analyse von Gehalten der Elemente durchgeführt wird, wobei zumindest ein Ist-Wert der Schmelzanalyse an ein Steuersystem geleitet wird, wobei zwischen Einfluss- und Kompensationselementen unterschieden wird, wobei Einflusselemente Elemente sind, welche einen Einfluss auf den Härteverlauf eines Endprodukts haben, und Kompensationselemente Elemente sind, die einen Einfluss auf den Härteverlauf haben und den Einfluss von Einflusselementen kompensieren, wobei die Größe des Einflusses des zumindest einen Ist-Werts der jeweiligen Einfluss- und Kompensationselemente auf den Härteverlauf des Endprodukts bekannt ist, wobei der zumindest eine Ist-Wert bezüglich derjenigen Einfluss- und Kompensationselemente erfasst wird, welche einen Einfluss auf den Härteverlauf des Endprodukts haben, wobei zum Härteverlauf des Endprodukts zumindest ein Kompensationselement ermittelt wird, welches den Härteverlauf einstellt, wobei eines oder mehrere Kompensationselemente in die Schmelze in einer Menge zulegiert werden, welche den Härteverlauf in einen Zielbereich bringen und den Einfluss eines oder mehrerer Einflusselemente auf den Härteverlauf kompensieren, und wobei hierfür im Steuersystem zwei Komponenten verwendet werden: ein Prognosemodell und ein Korrekturmodell, wobei mit dem Prognosemodell der Einfluss der Einfluss- und Kompensationselemente auf den Härteverlauf des Endprodukts und mit dem Korrekturmodell korrigierte Zielbereiche der Kompensationselemente berechnet werden, um den Härteverlauf in einen Zielbereich zu bringen, wobei zumindest ein Prognosemodell und/oder Korrekturmodell unter Anwendung einer multiplen linearen Regression für einen stabilen Härteverlauf erzeugt wird, wobei für die Erstellung des zumindest einen Prognosemodells und/oder Korrekturmodells zunächst ein Trainingsdatensatz basierend auf einer Ermittlung der Härtbarkeit von Stahl durch EN ISO 642 erstellt wird und hierauf basierend zumindest ein Prognosemodell und/oder Korrekturmodell für die spezifizierte Stabstahlgüte erzeugt wird.

Die Menge der zulegierten Kompensationselemente kann im Sinne der Erfindung auch 0 betragen, wenn dies für die Kompensation der Wirkung bzw. des Einflusses der Einflusselemente für nötig erachtet wird.

Eine Weiterbildung sieht vor, dass der Härteverlaufbereich nach EN ISO 683 auf die Stabstahlgüte spezifiziert wird.

Eine Weiterbildung sieht vor, dass die Analyse mittels Probenentnahme nach EN ISO 14284 durchgeführt wird.

Eine Weiterbildung sieht vor, dass auf die Stahlherstellung folgende Nachbehandlungsschritte bis zum Erhalt des Endprodukts unverändert durchgeführt werden.

Eine Weiterbildung sieht vor, dass zumindest drei Prognosemodelle und/oder Korrekturmodelle basierend auf durch die Ziellegierung definierten Filtern erzeugt werden, wobei ein erstes Prognosemodell und/oder Korrekturmodell für Stabstähle mit geringen Kohlenstoff- und Chromgehalten und insbesondere für eine Analyse umfassend C ≤ 0,2 Gew.-% und Cr ≤ 1,3 Gew.-% verwendet wird, wobei ein zweites Prognosemodell und/oder Korrekturmodell für Stabstähle mit höheren Kohlenstoff-, Chrom- und Mangangehalten und insbesondere für eine Analyse umfassend C ≤ 0,9 Gew.-%, 0,8 Gew.-% ≤ Cr ≤ 2,3 Gew.-% und Mn ≤ 1,5 Gew.-% verwendet wird und wobei ein drittes Prognosemodell und/oder Korrekturmodell für Stabstähle mit erhöhten Borgehalten und insbesondere für eine Analyse umfassend B > 0,0008 Gew.-% verwendet wird, wobei zusätzlich ein Borpotential B_{P} berücksichtigt wird.

Eine Weiterbildung sieht vor, dass der zumindest eine erfasste Ist-Wert der Analyse unter Heranziehung des Mittelwerts und/oder der Summe der Quadratwurzeln der mittleren quadratischen Abweichungen von positionsabhängigen Härtewerten HRCₙ dem zumindest einen Prognosemodell und/oder Korrekturmodell zugeordnet wird, wobei für den zumindest einen Ist-Wert der Mittelwert und/oder die Summe der Quadratwurzeln der mittleren quadratischen Abweichungen der positionsabhängigen Härtewerte HRCₙ für alle Prognosemodelle und/oder Korrekturmodelle ermittelt wird und basierend auf den Ergebnissen jenes Prognosemodell und/oder Korrekturmodell mit der höchsten Genauigkeit gewählt wird. Eine Weiterbildung sieht vor, dass zumindest ein weiteres Prognosemodell und/oder Korrekturmodell mittels der nachfolgenden Schritte erstellt wird:
i) Clusteranalyse mittels einer, mehrerer oder aller der folgenden Methoden: hierarchisches Clustering, partitionierendes Clustering, dichtebasierendes Clustering, gitterbasierendes Clustering;
ii) Bestimmung der optimalen Anzahl der Cluster mittels einer, mehrere oder aller der folgenden Methoden: Ellenbogenmethode, Silhouetten-Analyse, Entropiemethode, Intra-Cluster-Varianz, Gap-Statistik, Davies-Bouldin-Index, Calinski-Harabasz-Index, Akaike-Informationskriterium, Bayesianisches Informationskriterium, Hierarchische Clusteranalyse mit Dendrogramm, Gaussian Mixture Models;
iii) Optimierung, insbesondere mittels Methode kleinster Quadrate zur Bestimmung von Parametern Kₙ und k_{i,n} der multiplen linearen Regression, wobei Kₙ ein Grundhärtewert für eine Stabstahlgüte im Abstand n von der Oberfläche und k_{i,n} ein Gewichtungsfaktor, der den Einfluss eines bestimmten Elements auf die Härte im Abstand n von der Oberfläche beschreibt, ist.

Eine Weiterbildung sieht vor, dass zumindest ein weiteres Prognosemodell und/oder Korrekturmodell mittels der nachfolgenden Schritte erstellt wird:
i) Clusteranalyse mittels hierarchischem Clustering, partitionierendem Clustering, dichtebasierendem Clustering und/oder gitterbasierendem Clustering;
ii) Bestimmung der optimalen Anzahl der Cluster mittels Ellenbogenmethode, Silhouetten-Analyse, Entropiemethode, Intra-Cluster-Varianz, Gap-Statistik, Davies-Bouldin-Index, Calinski-Harabasz-Index, Akaike-Informationskriterium, Bayesianisches Informationskriterium, Hierarchische Clusteranalyse mit Dendrogramm und/oder Gaussian Mixture Models;
iii) Optimierung, insbesondere mittels Methode kleinster Quadrate zur Bestimmung von Parametern Kₙ und k_{i,n} der multiplen linearen Regression, wobei Kₙ ein Grundhärtewert für eine Stabstahlgüte im Abstand n von der Oberfläche und k_{i,n} ein Gewichtungsfaktor, der den Einfluss eines bestimmten Elements auf die Härte im Abstand n von der Oberfläche beschreibt, ist.

Eine Weiterbildung sieht vor, dass die Clusteranalyse zentroidbasiert ist und mittels nicht hierarchischem, partitionierendem Clustering, insbesondere K-Means-Methode durchgeführt wird.

Eine Weiterbildung sieht vor, dass die Bestimmung der optimalen Anzahl an Cluster mittels der Silhouetten- oder Ellenbogenmethode, insbesondere der Silhouetten-Methode durchgeführt wird.

Eine Weiterbildung sieht vor, dass im Rahmen der K-Means-Methode ein Proximitätsmaß zur Kategorisierung der Objekte hinsichtlich eines Merkmals Distanz oder Ähnlichkeit, insbesondere des Merkmals Distanz verwendet wird.

Eine Weiterbildung sieht vor, dass bei metrischen Variablen eine euklidische Distanz, quadrierte euklidische Distanz oder L1-Distanz, insbesondere die euklidische Distanz verwendet wird.

Eine Weiterbildung sieht vor, dass das Borpotential B_{P} zur Wirkung von Bor folgendermaßen bestimmt wird: ${B}_{p} = Gew . - {%}_{B} - \frac{{M}_{B}}{{M}_{N}} \times \left(Gew.-{%}_{N}-\frac{{M}_{N}}{{M}_{Ti}}\times Gew.-{%}_{Ti}\right) ,$ wobei
- Gew.-%ᵢ: der Gehalt des jeweiligen Elements i und
- Mᵢ: die molare Masse des jeweiligen Elements i in g/mol ist.

Eine Weiterbildung sieht vor, dass unter Heranziehung des zumindest einen Prognosemodells und/oder Korrekturmodells die Parameter Kₙ und k_{i,n} als Ausgangspunkte gewählt werden.

Eine Weiterbildung sieht vor, dass zumindest ein positionsabhängiger Härtewert HRCₙ an einer bestimmten Position n innerhalb des Härteverlaufs mithilfe der folgenden Formel ermittelt wird: ${HRC}_{n} = {K}_{n} + {\sum }_{i = 1}^{m} {k}_{i , n} ∗ Gew . - {%}_{i} ,$ wobei
- n: der Abstand des Härtewertes von der Oberfläche in mm bis in eine Tiefe von 50 mm,
- Kₙ: der Grundhärtewert im Abstand n von der Oberfläche,
- k_{i,n}: der Gewichtungsfaktor, der den Einfluss eines bestimmten Elements i auf die Härte im Abstand n von der Oberfläche wiedergibt,
- m: die Anzahl der berücksichtigten Elemente, und
- Gew.-%ᵢ: der Gehalt des jeweiligen Elements i ist.

Eine Weiterbildung sieht vor, dass bei der Erzeugung des zumindest einen Prognosemodells und/oder Korrekturmodells und der Ermittlung des Härtewerts HRCₙ als Elemente eines, mehrere oder alle aus der Gruppe von C, Si, Mn, P, S, Cu, Cr, Ni, Mo, V, Ti, Al, B, Nb, W, N, Co und Pb berücksichtigt werden.

Eine Weiterbildung sieht vor, dass bei der Erzeugung des zumindest einen Prognosemodells und/oder Korrekturmodells und der Ermittlung des Härtewerts HRCₙ als Elemente C, Si, Mn, P, S, Cu, Cr, Ni, Mo, V, Ti, Al, B, Nb, W, N, Co und/oder Pb berücksichtigt werden.

Eine Weiterbildung sieht vor, dass die Einflusselemente eines, mehrere oder alle Elemente aus der Gruppe Cu, Mo, Sn, Ni umfassen und die übrigen Elemente Kompensationselemente sind.

Eine Weiterbildung sieht vor, dass die Einflusselemente Cu, Mo, Sn und/oder Ni umfassen und die übrigen Elemente Kompensationselemente sind.

Eine Weiterbildung sieht vor, dass bei Ni-legierten Stabstahlgüten Cu, Mo, Sn, bei Mo-legierten Stabstahlgüten Cu, Sn, Ni und bei Ni-Mo-legierten Stabstahlgüten Cu, Sn Einflusselemente sind, wobei in diesen Fällen entsprechend die zuvor genannten Legierungselemente auch als Kompensationselemente geeignet sind.

Eine Weiterbildung sieht vor, dass die Analyse zumindest in der ersten Schmelze des letzten primärmetallurgischen Aggregates einer Erzeugungsroute, insbesondere ab einem Abstich von Rohstahl, vor oder nach einer ersten Zugabe von Legierungselementen und zumindest nach einer zweiten Zugabe von Legierungselementen durchgeführt wird.

Eine Weiterbildung sieht vor, dass, wenn ein erstes Element durch einen Einsatzstoff in einem Anteil eingebracht wird, der unterhalb eines gewünschten, durch die Ziellegierung definierten Zielbereichs liegt, das erste Element, sofern es ein gewünschtes Legierungselement ist, in dem Umfang zulegiert wird, der zum Erreichen des Zielbereichs notwendig ist, oder, wenn das erste Element durch einen Einsatzstoff in einem Anteil eingebracht wird, der bereits im Zielbereich entspricht, das erste Element nicht mehr zulegiert wird oder, wenn das erste Element durch einen Einsatzstoff in einem Anteil eingebracht wird, der über dem Zielbereich liegt, das erste Element durch einen verringerten Legierungsanteil eines zweiten ähnlich wirkenden Elements oder einen erhöhten Legierungsanteil eines gegenläufig wirkenden dritten Elements kompensiert wird.

Eine Weiterbildung sieht vor, dass das verwendete Korrekturmodell eine Reihung der unterschiedlichen Kompensationselemente nach auflösungs- und ausbringungsspezifischen sowie ökonomischen Gesichtspunkten festlegt.

Der Begriff "Auflösung" bezieht sich auf die Auflösung der Einsatzstoffe in der Schmelze. Dies bedeutet in diesem Zusammenhang, dass im Zweifel ein Element als Kompensationselement eher nicht berücksichtigt wird, wenn sein Auflösen in der Schmelze zu lange dauert und insbesondere den gesamten Prozess verzögern würde.

Der Begriff "Ausbringung" bezieht sich auf die Ausbeute.

Eine Weiterbildung sieht vor, dass die Ziellegierung die folgende Ziellegierungszusammensetzung in Gew.-% umfasst:

| | |
|---|---|
| C | 0,05-1,3 |
| Si | 0,05-1,2 |
| Mn | 0,1-2,0 |

zudem Eisen und unvermeidbare Verunreinigungen,
wobei lediglich optional eines, mehrere oder alle der folgenden Elemente in Gew.-% enthalten sind:

| | |
|---|---|
| Cr | 0,001-2,0 |
| Cu | 0,001-0,4 |
| Mo | 0,001-1,0 |
| Ni | 0,001-2,5 |
| V | 0,001-0,4 |
| Sn | 0,001-0,03 |
| B | 0,0005-0,008 |
| N | 0,0001-0,02 |
| Ti | 0,001-0,04 |
| Al | 0,0005-0,05 |
| Nb | 0,001-0,1 |
| Co | 0,001-0,2 |
| Pb | 0,001-0,4 |
| W | 0,001-0,5 |
| P | ≤0,045 |
| S | ≤ 0,06. |

Eine Weiterbildung sieht vor, dass die Ziellegierung die folgende Ziellegierungszusammensetzung in Gew.-% umfasst:

| | |
|---|---|
| C | 0,05-1,3 |
| Si | 0,05-1,2 |
| Mn | 0,1-2,0 |

zudem Eisen und unvermeidbare Verunreinigungen,
wobei lediglich optional folgende Elemente in Gew.-% enthalten sind:

| | |
|---|---|
| Cr | 0,001-2,0 |
| Cu | 0,001-0,4 |
| Mo | 0,001-1,0 |
| Ni | 0,001-2,5 |
| V | 0,001-0,4 |
| Sn | 0,001-0,03 |
| B | 0,0005-0,008 |
| N | 0,0001-0,02 |
| Ti | 0,001-0,04 |
| Al | 0,0005-0,05 |
| Nb | 0,001-0,1 |
| Co | 0,001-0,2 |
| Pb | 0,001-0,4 |
| W | 0,001-0,5 |
| P | ≤0,045 und/oder |
| S | ≤ 0,06. |

Ferner betrifft die Erfindung ein Steuersystem zur Herstellung von Stabstählen mit einem auf eine Stabstahlgüte spezifizierten Härteverlaufbereich, welcher durch eine Ziellegierung sichergestellt wird, und zur Durchführung des erfindungsgemäßen Verfahrens, wobei das Steuersystem zwei Komponenten umfasst: Ein Prognosemodell und ein Korrekturmodell, wobei das Prognosemodell dazu ausgelegt ist, eine Wirkung von Einflusselementen und Kompensationselementen auf den Härteverlauf des Stabstahls zu berechnen, und das Korrekturmodell dazu ausgelegt ist, Zielwerte von Kompensationselementen zu berechnen, um den Härteverlauf in einen Zielbereich zu bringen.

Eine Weiterbildung sieht vor, dass zumindest drei Prognosemodelle und/oder Korrekturmodelle basierend auf durch die Ziellegierung definierten Filtern erzeugt werden, wobei ein erstes Prognosemodell und/oder Korrekturmodell für Stabstähle mit geringen Kohlenstoff- und Chromgehalten und insbesondere für eine Analyse umfassend C ≤ 0,2 Gew.-% und Cr ≤ 1,3 Gew.-% verwendet wird, wobei ein zweites Prognosemodell und/oder Korrekturmodell für Stabstähle mit höheren Kohlenstoff-, Chrom- und Mangangehalten und insbesondere für eine Analyse umfassend C ≤ 0,9 Gew.-%, 0,8 Gew.-% ≤ Cr ≤ 2,3 Gew.-% und Mn ≤ 1,5 Gew.-% verwendet wird und wobei ein drittes Prognosemodell und/oder Korrekturmodell für Stabstähle mit erhöhten Borgehalten und insbesondere für eine Analyse umfassend B > 0,0008 Gew.-% verwendet wird, wobei zusätzlich ein Borpotential B_{P} berücksichtigt wird.

Eine Weiterbildung sieht vor, dass zumindest ein weiteres Prognosemodell und/oder Korrekturmodell mittels der nachfolgenden Schritte erstellt wird:
i) Clusteranalyse mittels einer, mehrerer oder aller der folgenden Methoden: hierarchisches Clustering, partitionierendes Clustering, dichtebasierendes Clustering, gitterbasierendes Clustering;
ii) Bestimmung der optimalen Anzahl der Cluster mittels einer, mehrere oder aller der folgenden Methoden: Ellenbogenmethode, Silhouetten-Analyse, Entropiemethode, Intra-Cluster-Varianz, Gap-Statistik, Davies-Bouldin-Index, Calinski-Harabasz-Index, Akaike-Informationskriterium, Bayesianisches Informationskriterium, Hierarchische Clusteranalyse mit Dendrogramm, Gaussian Mixture Models;
iii) Optimierung, insbesondere mittels Methode kleinster Quadrate zur Bestimmung von Parametern Kₙ und k_{i,n} der multiplen linearen Regression, wobei Kₙ ein Grundhärtewert im Abstand n von der Oberfläche für eine Stabstahlgüte und k_{i,n} ein Gewichtungsfaktor, der den Einfluss eines bestimmten Elements auf die Härte im Abstand n von der Oberfläche beschreibt, ist.

Eine Weiterbildung sieht vor, dass zumindest ein weiteres Prognosemodell und/oder Korrekturmodell mittels der nachfolgenden Schritte erstellt wird:
i) Clusteranalyse mittels hierarchischem Clustering, partitionierendem Clustering, dichtebasierendem Clustering und/oder gitterbasierendem Clustering;
ii) Bestimmung der optimalen Anzahl der Cluster mittels Ellenbogenmethode, Silhouetten-Analyse, Entropiemethode, Intra-Cluster-Varianz, Gap-Statistik, Davies-Bouldin-Index, Calinski-Harabasz-Index, Akaike-Informationskriterium, Bayesianisches Informationskriterium, Hierarchische Clusteranalyse mit Dendrogramm und/oder Gaussian Mixture Models;
iii) Optimierung, insbesondere mittels Methode kleinster Quadrate zur Bestimmung von Parametern Kₙ und k_{i,n} der multiplen linearen Regression, wobei Kₙ ein Grundhärtewert für eine Stabstahlgüte im Abstand n von der Oberfläche und k_{i,n} ein Gewichtungsfaktor, der den Einfluss eines bestimmten Elements auf die Härte im Abstand n von der Oberfläche beschreibt, ist.

Eine Weiterbildung sieht vor, dass das Borpotential B_{P} zur Wirkung von Bor folgendermaßen bestimmbar ist: ${B}_{p} = Gew . - {%}_{B} - \frac{{M}_{B}}{{M}_{N}} \times \left(Gew.-{%}_{N}-\frac{{M}_{N}}{{M}_{Ti}}\times Gew.-{%}_{Ti}\right) ,$ wobei
- Gew.-%ᵢ: der Gehalt des jeweiligen Elements i und
- Mᵢ: die molare Masse des jeweiligen Elements i in g/mol ist.

Eine Weiterbildung sieht vor, dass zumindest ein positionsabhängiger Härtewert HRCₙ an einer bestimmten Position n innerhalb des Härteverlaufs mithilfe der folgenden Formel ermittelbar ist: ${HRC}_{n} = {K}_{n} + {\sum }_{i = 1}^{m} {k}_{i , n} ∗ Gew . - {%}_{i} ,$ wobei
- n: der Abstand des Härtewertes von der Oberfläche in mm bis in eine Tiefe von 50 mm,
- Kₙ: der Grundhärtewert im Abstand n von der Oberfläche,
- k_{i,n}: der Gewichtungsfaktor, der den Einfluss eines bestimmten Elements i auf die Härte im Abstand n von der Oberfläche wiedergibt,
- m: die Anzahl der berücksichtigten Elemente, und
- Gew.-%ᵢ: der Gehalt des jeweiligen Elements i ist.

Eine Weiterbildung sieht vor, dass die Ziellegierung die folgende Ziellegierungszusammensetzung in Gew.-% umfasst:

| | |
|---|---|
| C | 0,05-1,3 |
| Si | 0,05-1,2 |
| Mn | 0,1-2,0 |

zudem Eisen und unvermeidbare Verunreinigungen,
wobei lediglich optional eines, mehrere oder alle der folgenden Elemente in Gew.-% enthalten sind:

| | |
|---|---|
| Cr | 0,001-2,0 |
| Cu | 0,001-0,4 |
| Mo | 0,001-1,0 |
| Ni | 0,001-2,5 |
| V | 0,001-0,4 |
| Sn | 0,001-0,03 |
| B | 0,0005-0,008 |
| N | 0,0001-0,02 |
| Ti | 0,001-0,04 |
| Al | 0,0005-0,05 |
| Nb | 0,001-0,1 |
| Co | 0,001-0,2 |
| Pb | 0,001-0,4 |
| W | 0,001-0,5 |
| P | ≤0,045 |
| S | ≤ 0,06. |

Eine Weiterbildung sieht vor, dass die Ziellegierung die folgende Ziellegierungszusammensetzung in Gew.-% umfasst:

| | |
|---|---|
| C | 0,05-1,3 |
| Si | 0,05-1,2 |
| Mn | 0,1-2,0 |

zudem Eisen und unvermeidbare Verunreinigungen,
wobei lediglich optional folgenden Elemente in Gew.-% enthalten sind:

| | |
|---|---|
| Cr | 0,001-2,0 |
| Cu | 0,001-0,4 |
| Mo | 0,001-1,0 |
| Ni | 0,001-2,5 |
| V | 0,001-0,4 |
| Sn | 0,001-0,03 |
| B | 0,0005-0,008 |
| N | 0,0001-0,02 |
| Ti | 0,001-0,04 |
| Al | 0,0005-0,05 |
| Nb | 0,001-0,1 |
| Co | 0,001-0,2 |
| Pb | 0,001-0,4 |
| W | 0,001-0,5 |
| P | ≤0,045 und/oder |
| S | ≤ 0,06. |

Ferner betrifft die Erfindung einen Stabstahl für Wärmebehandlungsanwendungen, hergestellt nach dem erfindungsgemäßen Verfahren.

Die Erfindung wird beispielhaft anhand einer Zeichnung dargestellt. Es zeigen dabei:
- Figur 1: den Prozessablauf des dynamischen Legierens;
- Figur 2: das Ergebnis einer beispielhaften Durchführung der RMSE-Methode zur Zuordnung des Analyse-Ist-Wert zu einem Cluster/Prognosemodell;
- Figur 3: beispielhafte Härtewertvorhersagen/Jominy-Messpunkte in Abhängigkeit des gewählten Modells und deren Abweichungen zu den gemessenen Werten;
- Figur 4: beispielhafte Elementgehalte einer Stabstahllegierung;
- Figur 5: die tabellarische Zusammenfassung der Elementgehalte in einem Stahl 1858 nach Zugabe von Schrott mit niedriger Einflusselementfracht, nach Zugabe von Schrott mit hoher Einflusselementfracht, nach Zugabe von Schrott mit hoher Einflusselementfracht und dynamischen Legieren;
- Figur 6: den berechneten Härteverlauf des Stahls 1858 nach Zugabe von Schrott mit niedriger Einflusselementfracht in Relation zum durch die gewünschte Stabstahlgüte spezifizierten Härteverlaufbereich;
- Figur 7: den berechneten Härteverlauf des Stahls 1858 nach Zugabe von Schrott mit niedriger Einflusselementfracht und des Stahls 1858 nach Zugabe von Schrott mit hoher Einflusselementfracht in Relation zum durch die gewünschte Stabstahlgüte spezifizierten Härteverlaufbereich;
- Figur 8: den berechneten Härteverlauf des Stahls 1858 nach Zugabe von Schrott mit niedriger Einflusselementfracht und des Stahls 1858 nach Zugabe von Schrott mit hoher Einflusselementfracht und dynamischen Legieren in Relation zum durch die gewünschte Stabstahlgüte spezifizierten Härteverlaufbereich.

Die Erfinder haben erkannt, dass es besonders vorteilhaft ist, zwischen Einfluss- und Kompensationselementen zu unterscheiden.

Die Wirkung bzw. der Einfluss der Einflusselemente wird durch das Zulegieren von Kompensationselementen kompensiert.

Die Wirkung bzw. der Einfluss, der in der Schmelze bereits vorhandenen Einflusselemente auf den Härteverlauf des Stabstahlprodukts wird mit einem Prognosemodell modelliert. Eine gegebenenfalls notwendige Korrektur wird mit einem Korrekturmodell ermittelt.

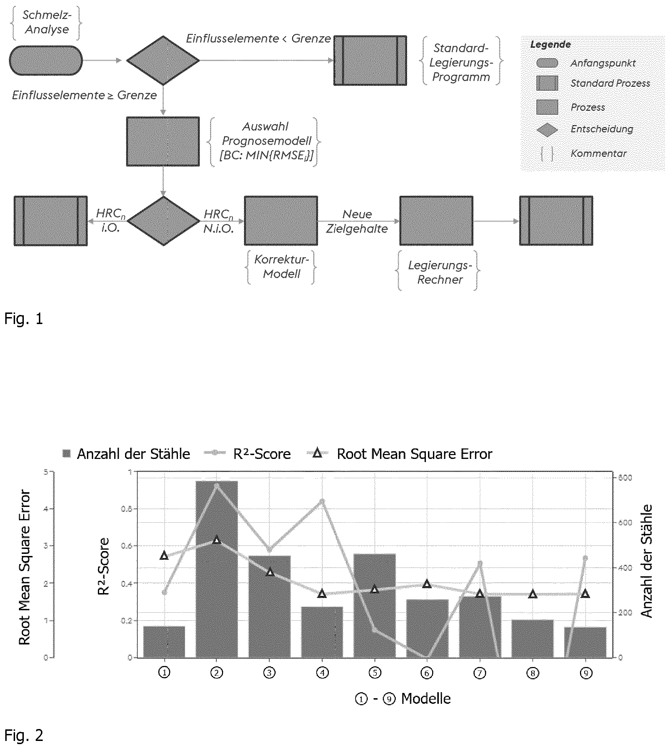

Figur 1 illustriert in vereinfachter Art und Weise das erfindungsgemäße Verfahren. Ausgehend von einer Schmelzanlayse wird entschieden, ob die Analyse innerhalb der spezifizierten Grenzen für die Einflusselementgehalte liegt oder sich davon unterscheidet. Wenn die Einflusselementgehalte innerhalb der Grenzen liegen, wird der Standard-Prozess durchgeführt, d.h. es kommt nicht zum dynamischen Legieren. Liegen die Einflusselementgehalte jedoch außerhalb der spezifizierten Grenzen, wird mittels des Prognosemodells der Einfluss auf den Härteverlauf ermittelt, um festzustellen, ob der prognostizierte Härteverlauf innerhalb oder außerhalb des durch die Zielstabstahlgüte definierten Härteverlaufbereichs liegt. Liegt der Härteverlauf im spezifizierten Bereich, wird der Standard-Prozess durchgeführt, d.h. es kommt nicht zum dynamischen Legieren. Wenn der prognostizierte Härteverlauf jedoch außerhalb des spezifizierten Bereichs liegt, werden mithilfe des Korrekturmodells adaptierte Legierungselementgehalte berechnet, sodass durch entsprechende Zugabe von Kompensationselementen die Wirkung der Einflusselemente dahingehend kompensiert werden kann, dass der Härteverlauf in den spezifizierten Bereich gebracht wird. Anschließend wird wieder nach dem Standard-Legierungsprozess verfahren. Nach dem dynamischen Legieren können gegebenenfalls auch erneut Analyse-Ist-Werte in das Prognosemodell eingespeist werden. Liegt der prognostizierte Härteverlauf im spezifizierten Zielbereich, wird nachfolgend nach dem Standard-Prozess verfahren. Liegt der prognostizierte Härteverlauf jedoch immer noch nicht im spezifizierten Zielbereich, kann erneut dynamisches Legieren durchgeführt werden.

Eine Silhouettenanalyse kann zur Untersuchung des Trennungsabstands zwischen den resultierenden Clustern verwendet werden. Die Silhouettengrafik zeigt, wie nahe jeder Punkt eines Clusters an den Punkten der benachbarten Cluster liegt und bietet somit eine Möglichkeit zur visuellen Bewertung von Parametern wie der Anzahl der Cluster. Dieses Maß hat einen Bereich von [-1, 1]. Silhouettenkoeffizienten nahe +1 zeigen an, dass die Probe weit von den benachbarten Clustern entfernt ist. Ein Wert von 0 zeigt an, dass sich die Probe auf oder sehr nahe an der Entscheidungsgrenze zwischen zwei benachbarten Clustern befindet, und negative Werte bedeuten, dass diese Proben möglicherweise dem falschen Cluster zugeordnet wurden. Auch anhand der Ausdehnung der Silhouettengrafiken lässt sich die Größe des Clusters ablesen.

Ergebnisse einer Silhouettenanalyse für einen Beispieldatensatz sind beispielsweise auf der folgenden Webseite gezeigt:
https://scikit-learn.org/1.5/auto examples/cluster/plot kmeans silhouette analysis.html

Die Analyse wurde dabei unter Annahme unterschiedlicher Clusteranzahlen (n=2-6) durchgeführt. Die resultierenden Silhouettengrafiken zeigen entsprechend 2-6 unterschiedlich ausgedehnte Cluster. Die Größen der Cluster sollten annähernd gleichverteilt vorliegen, wie dies bei dem Beispiel mit vier Clustern der Fall ist.

Zur Auswahl des Prognosemodells (HRC-Prädiktionsmodells) wird der zumindest eine aus der Schmelze bestimmte Analysen-Ist-Wert in jedes der Prognosemodelle eingespeist, und der Mittelwert und/oder die Summe der Quadratwurzeln der mittleren quadratischen Abweichungen (RMSE) der Härtewerte je Abstand n von der Oberfläche und/oder das Bestimmtheitsmaß (R²-Score) werden bestimmt (Figur 2).

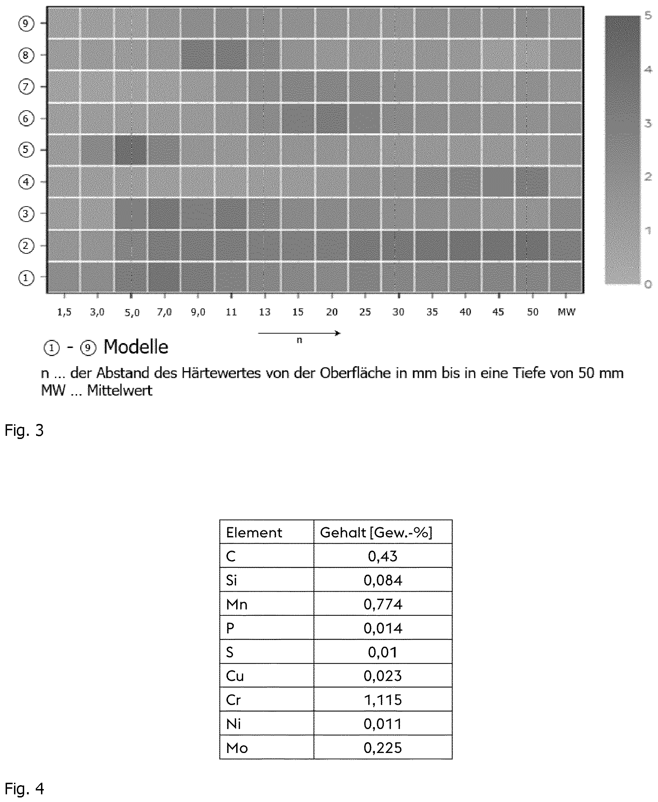

Bei der Quadratwurzel der mittleren quadratischen Abweichung handelt es sich um ein Maß für die Unterschiede zwischen vorhergesagten Werten (vgl. Härtewertvorhersagen bzw. Jominymesspunkte in Figur 3) einerseits und gemessenen Werten andererseits. Die Abweichung ist normalerweise einfach eine Differenz von Skalaren.

Alternativ kann auch das Bestimmtheitsmaß (R²-Score) als Bewertungskriterium herangezogen werden. Unter R² versteht man in der Statistik eine Kennzahl zur Beurteilung der Anpassungsgüte einer Regression. Das Bestimmtheitsmaß beruht auf der Quadratsummenzerlegung, bei der die totale Quadratsumme, in die durch das Regressionsmodell erklärte Quadratsumme einerseits und die Residuenquadratsumme andererseits zerlegt wird.

Das Prognosemodell, welches sich durch den geringsten RMSE-Wert bzw. den höchsten R²-Score auszeichnet, wird schließlich zur Modellierung des Härteverlaufs herangezogen.

Für die Berechnung des multiplen, linearen Regressionsmodells für die prädiktiven Härtewerte wird die folgende Formel verwendet: ${HRC}_{n} = {K}_{n} + {\sum }_{i = 1}^{m} {k}_{i , n} ∗ Gew . - {%}_{i} ,$ wobei
- n: der Abstand des Härtewertes von der Oberfläche in mm bis in eine Tiefe von 50 mm,
- Kₙ: der Grundhärtewert im Anstand n von der Oberfläche,
- k_{i,n}: der Gewichtungsfaktor, der den Einfluss eines bestimmten Elements i auf die Härte im Abstand n von der Oberfläche wiedergibt,
- m: die Anzahl der berücksichtigten Elemente, und
- Gew.-%ᵢ: der Gehalt des jeweiligen Elements i ist.

Hierbei wird der Härtewert für einen bestimmten Abstand n von der Oberfläche in mm berechnet (vgl. SEP 1664), wobei die Elemente C, Si, Mn, P, S, Cu, Cr, Ni, Mo, V, Ti, Al, B, Nb, W, N, Co und Pb berücksichtigt werden.

Für eine beispielhafte Stahllegierung (Figur 4) ergibt sich für die Berechnung des Härtewertes bei Position 1,5 mm und 3 mm folgendes: HRC1,5mm = 34,13C * Gew. -%C - 0,53Si * Gew. -%Si - 0,72Mn * Gew. -%Mn + 2,21Cr * Gew. -%Cr + 29,77S * Gew. -%S - 16,79P * Gew. -%P - 10,46Mo * Gew. -%Mo - 17,21Cu * Gew. -%Cu + 2,56Ni * Gew. -%Ni + 44,03; HRC3mm = 36,77C * Gew. -%C - 0,42Si * Gew. -%Si + 0,12Mn * Gew. -%Mn+2,41Cr * Gew. -%Cr - 31,86S * Gew. -%S - 15,02P * Gew. -%P - 7,54Mo * Gew. -%Mo - 18,54Cu * Gew. -%Cu + 2,53Ni * Gew. -%Ni + 42.

Es handelt sich hierbei lediglich um eine beispielhafte Berechnung zur Veranschaulichung der Härtewertberechnung, in dem aus Gründen der Übersichtlichkeit nicht alle möglichen Elemente betrachtet werden. Dies ist in keiner Weise als einschränkend zu verstehen; es können auch andere bzw. weitere Elemente in die Berechnung einfließen.

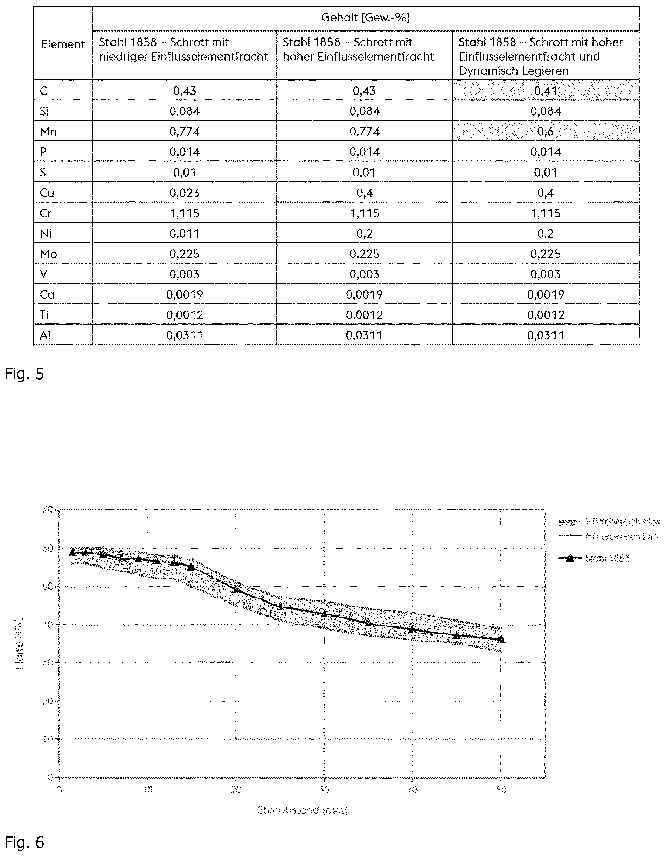

Figur 5 zeigt drei Legierungszusammensetzungen eines Stahls 1858:
1) Schrott mit niedriger Einflusselementfracht (LD-Stahl 42CrMo4);
2) Schrott mit hoher Einflusselementfracht (simulierte EAF-Werkstoffzusammensetzung);
3) Schrott mit hoher Einflusselementfracht und dynamischen Legieren.

In Figur 6 ist der gemessen Härteverlauf des LD-Stahls 42CrMo4 zu sehen. Zudem sind die zulässigen Härtegrenzwerte, welche einen angestrebten Härteverlaufbereich (Zielbereich) aufspannen gezeigt. Wie man erkennen kann, liegen die gemessenen Härtewerte innerhalb des Zielbereichs.

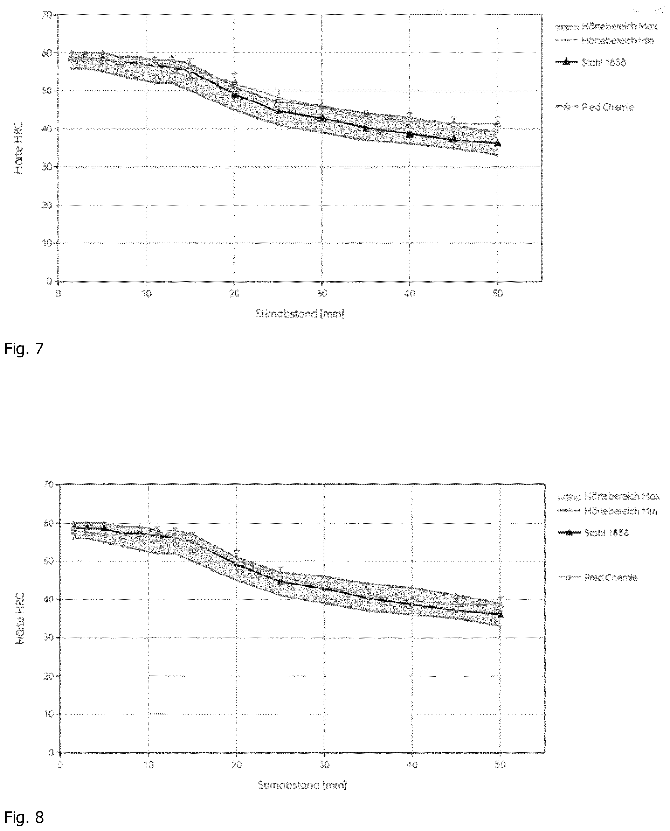

Figur 7 stellt dem Härteverlauf des LD-Stahls einen Härteverlauf eines EAF-Stahls mit höherer Einflusselementfracht gegenüber. Wie deutlich zu erkennen ist, erfüllt diese Legierung nun nicht mehr in allen Abständen die spezifizierten Härtekriterien und ist somit nicht mehr normkonform.

Um dennoch einen Stabstahl mit einem auf eine Stabstahlgüte spezifizierten Härteverlaufbereich herstellen zu können, müssen Legierungselemente angepasst werden, d.h. die Wirkung der Einflusselemente muss durch Kompensationselemente kompensiert werden. Dafür wird zunächst durch das Prognosemodell der Einfluss auf den Härteverlauf prognostiziert. Anschließend werden durch das Korrekturmodell adaptierte Gehalte der Elemente C, Si, Mn und Cr berechnet. Letzteres erfolgt unter Berücksichtigung der chemischen Grenzwerte der angestrebten Stabstahlgüte. Dies hat zur Folge, dass beispielsweise der Härteverlauf des Stahls 1858 nach dem dynamischen Legieren im durch die jeweilige Stabstahlgüte definierten Härteverlaufbereich liegen würde (Figur 8). Darüber hinaus können die Legierungskosten (vgl. C- und Mn-Gehalt) gesenkt werden.

Mit dem vorliegenden Verfahren gelingt somit ein dynamisches und flexibles Legieren, bei welchem der Einfluss bzw. die Wirkung der Einflusselemente durch eine gezielte Zugabe der Kompensationselemente kompensiert wird.

Die Kompensationselemente können u.a. unter Berücksichtigung auflösungs- und ausbringungsspezifischer sowie ökonomischer Aspekte ausgewählt werden, sodass hierdurch die Kosteneffizienz des Gesamtverfahrens deutlich erhöht wird.

Außerdem gelingt es mit dem vorliegenden Verfahren, die gewünschten werkstoffkundlichen Eigenschaften trotzt Verwendung von Einsatzstoffen mit erhöhter Einflusselementfracht sicher einzuhalten.

## Patentansprüche

1. Verfahren zur Herstellung von Stabstählen mit einem auf eine Stabstahlgüte spezifizierten Härteverlaufbereich, welcher durch eine Ziellegierung sichergestellt wird, wobei zumindest ein primärmetallurgisches Aggregat zu einer ersten Schmelze führt, wobei aus der ersten Schmelze zumindest eine Analyse von Gehalten der Elemente durchgeführt wird, wobei zumindest ein Ist-Wert der Schmelzanalyse an ein Steuersystem geleitet wird,
**dadurch gekennzeichnet, dass**
zwischen Einfluss- und Kompensationselementen unterschieden wird, wobei
Einflusselemente Elemente sind, welche einen Einfluss auf den Härteverlauf eines Endprodukts haben, und Kompensationselemente Elemente sind, die einen Einfluss auf den Härteverlauf haben und den Einfluss von Einflusselementen kompensieren, wobei
die Größe des Einflusses des zumindest einen Ist-Werts der jeweiligen Einfluss- und Kompensationselemente auf den Härteverlauf des Endprodukts bekannt ist, wobei
der zumindest eine Ist-Wert bezüglich derjenigen Einfluss- und Kompensationselemente erfasst wird, welche einen Einfluss auf den Härteverlauf des Endprodukts haben, wobei
zum Härteverlauf des Endprodukts zumindest ein Kompensationselement ermittelt wird, welches den Härteverlauf einstellt, wobei
eines oder mehrere Kompensationselemente in die Schmelze in einer Menge zulegiert werden, welche den Härteverlauf in einen Zielbereich bringen und den Einfluss eines oder mehrerer Einflusselemente auf den Härteverlauf kompensieren, und wobei
hierfür im Steuersystem zwei Komponenten verwendet werden: ein Prognosemodell und ein Korrekturmodell, wobei mit dem Prognosemodell der Einfluss der Einfluss- und Kompensationselemente auf den Härteverlauf des Endprodukts und mit dem Korrekturmodell korrigierte Zielbereiche der Kompensationselemente berechnet werden, um den Härteverlauf in einen Zielbereich zu bringen, wobei
zumindest ein Prognosemodell und/oder Korrekturmodell unter Anwendung einer multiplen linearen Regression für einen stabilen Härteverlauf erzeugt wird, wobei für die Erstellung des zumindest einen Prognosemodells und/oder Korrekturmodells zunächst ein Trainingsdatensatz basierend auf einer Ermittlung der Härtbarkeit von Stahl durch EN ISO 642 erstellt wird und hierauf basierend zumindest ein Prognosemodell und/oder Korrekturmodell für die spezifizierte Stabstahlgüte erzeugt wird.

2. Verfahren nach Anspruch 1, **dadurch gekennzeichnet, dass** der Härteverlaufbereich nach EN ISO 683 auf die Stabstahlgüte spezifiziert wird.

3. Verfahren nach Anspruch 1 oder 2, **dadurch gekennzeichnet, dass** die Analyse mittels Probenentnahme nach EN ISO 14284 durchgeführt wird.

4. Verfahren nach einem der vorhergehenden Ansprüche, **dadurch gekennzeichnet, dass** auf die Stahlherstellung folgende Nachbehandlungsschritte bis zum Erhalt des Endprodukts unverändert durchgeführt werden.

5. Verfahren nach einem der vorhergehenden Ansprüche, **dadurch gekennzeichnet, dass** zumindest drei Prognosemodelle und/oder Korrekturmodelle basierend auf durch die Ziellegierung definierten Filtern erzeugt werden, wobei ein erstes Prognosemodell und/oder Korrekturmodell für Stabstähle mit geringen Kohlenstoff- und Chromgehalten und insbesondere für eine Analyse umfassend C ≤ 0,2 Gew.-% und Cr ≤ 1,3 Gew.-% verwendet wird, wobei ein zweites Prognosemodell und/oder Korrekturmodell für Stabstähle mit höheren Kohlenstoff-, Chrom- und Mangangehalten und insbesondere für eine Analyse umfassend C ≤ 0,9 Gew.-%, 0,8 Gew.-% ≤ Cr ≤ 2,3 Gew.-% und Mn ≤ 1,5 Gew.-% verwendet wird und wobei ein drittes Prognosemodell und/oder Korrekturmodell für Stabstähle mit erhöhten Borgehalten und insbesondere für eine Analyse umfassend B > 0,0008 Gew.-% verwendet wird, wobei zusätzlich ein Borpotential B_{P} berücksichtigt wird.

6. Verfahren nach einem der vorhergehenden Ansprüche, **dadurch gekennzeichnet, dass** der zumindest eine erfasste Ist-Wert der Analyse unter Heranziehung des Mittelwerts und/oder der Summe der Quadratwurzeln der mittleren quadratischen Abweichungen von positionsabhängigen Härtewerten HRCₙ dem zumindest einen Prognosemodell und/oder Korrekturmodell zugeordnet wird, wobei für den zumindest einen Ist-Wert der Mittelwert und/oder die Summe der Quadratwurzeln der mittleren quadratischen Abweichungen der positionsabhängigen Härtewerte HRCₙ für alle Prognosemodelle und/oder Korrekturmodelle ermittelt wird und basierend auf den Ergebnissen jenes Prognosemodell und/oder Korrekturmodell mit der höchsten Genauigkeit gewählt wird.

7. Verfahren nach einem der vorhergehenden Ansprüche, **dadurch gekennzeichnet, dass** zumindest ein weiteres Prognosemodell und/oder Korrekturmodell mittels der nachfolgenden Schritte erstellt wird:
i) Clusteranalyse mittels einer, mehrerer oder aller der folgenden Methoden: hierarchisches Clustering, partitionierendes Clustering, dichtebasierendes Clustering, gitterbasierendes Clustering;
ii) Bestimmung der optimalen Anzahl der Cluster mittels einer, mehrere oder aller der folgenden Methoden: Ellenbogenmethode, Silhouetten-Analyse, Entropiemethode, Intra-Cluster-Varianz, Gap-Statistik, Davies-Bouldin-Index, Calinski-Harabasz-Index, Akaike-Informationskriterium, Bayesianisches Informationskriterium, Hierarchische Clusteranalyse mit Dendrogramm, Gaussian Mixture Models;
iii) Optimierung, insbesondere mittels Methode kleinster Quadrate zur Bestimmung von Parametern Kₙ und k_{i,n} der multiplen linearen Regression, wobei Kₙ ein Grundhärtewert für eine Stabstahlgüte im Abstand n von der Oberfläche und k_{i,n} ein Gewichtungsfaktor, der den Einfluss eines bestimmten Elements auf die Härte im Abstand n von der Oberfläche beschreibt, ist.

8. Verfahren nach Anspruch 7, **dadurch gekennzeichnet, dass** die Clusteranalyse zentroidbasiert ist und mittels nicht hierarchischem, partitionierendem Clustering, insbesondere K-Means-Methode durchgeführt wird.

9. Verfahren nach Anspruch 7, **dadurch gekennzeichnet, dass** die Bestimmung der optimalen Anzahl an Cluster mittels der Silhouetten- oder Ellenbogenmethode, insbesondere der Silhouetten-Methode durchgeführt wird.

10. Verfahren nach Anspruch 8, **dadurch gekennzeichnet, dass** im Rahmen der K-Means-Methode ein Proximitätsmaß zur Kategorisierung der Objekte hinsichtlich eines Merkmals Distanz oder Ähnlichkeit, insbesondere des Merkmals Distanz verwendet wird.

11. Verfahren nach Anspruch 10, **dadurch gekennzeichnet, dass** bei metrischen Variablen eine euklidische Distanz, quadrierte euklidische Distanz oder L1-Distanz, insbesondere die euklidische Distanz verwendet wird.

12. Verfahren nach Anspruch 5, **dadurch gekennzeichnet, dass** das Borpotential B_{P} zur Wirkung von Bor folgendermaßen bestimmt wird: ${B}_{p} = Gew . - {%}_{B} - \frac{{M}_{B}}{{M}_{N}} \times \left(Gew.-{%}_{N}-\frac{{M}_{N}}{{M}_{Ti}}\times Gew.-{%}_{Ti}\right) ,$ wobei
Gew.-%ᵢ der Gehalt des jeweiligen Elements i und
Mᵢ die molare Masse des jeweiligen Elements i in g/mol ist.

13. Verfahren nach einem der vorhergehenden Ansprüche, **dadurch gekennzeichnet, dass** unter Heranziehung des zumindest einen Prognosemodells und/oder Korrekturmodells die Parameter Kₙ und k_{i,n} als Ausgangspunkte gewählt werden.

14. Verfahren nach einem der vorhergehenden Ansprüche, **dadurch gekennzeichnet, dass** zumindest ein positionsabhängiger Härtewert HRCₙ an einer bestimmten Position n innerhalb des Härteverlaufs mithilfe der folgenden Formel ermittelt wird: ${HRC}_{n} = {K}_{n} + {\sum }_{i = 1}^{m} {k}_{i , n} ∗ Gew . - {%}_{i} ,$ wobei
n der Abstand des Härtewertes von der Oberfläche in mm bis in eine Tiefe von 50 mm,
Kₙ der Grundhärtewert im Abstand n von der Oberfläche,
k_{i,n} der Gewichtungsfaktor, der den Einfluss eines bestimmten Elements i auf die Härte im Abstand n von der Oberfläche wiedergibt,
m die Anzahl der berücksichtigten Elemente, und
Gew.-%ᵢ der Gehalt des jeweiligen Elements i ist.

15. Verfahren nach einem der vorhergehenden Ansprüche, **dadurch gekennzeichnet, dass** bei der Erzeugung des zumindest einen Prognosemodells und/oder Korrekturmodells und der Ermittlung des Härtewerts HRCₙ als Elemente eines, mehrere oder alle aus der Gruppe von C, Si, Mn, P, S, Cu, Cr, Ni, Mo, V, Ti, Al, B, Nb, W, N, Co und Pb berücksichtigt werden.

16. Verfahren nach Anspruch 15, **dadurch gekennzeichnet, dass** die Einflusselemente eines, mehrere oder alle Elemente aus der Gruppe Cu, Mo, Sn, Ni umfassen und die übrigen Elemente Kompensationselemente sind.

17. Verfahren nach Anspruch 16, **dadurch gekennzeichnet, dass** bei Ni-legierten Stabstahlgüten Cu, Mo, Sn, bei Mo-legierten Stabstahlgüten Cu, Sn, Ni und bei Ni-Mo-legierten Stabstahlgüten Cu, Sn Einflusselemente sind, wobei in diesen Fällen entsprechend die zuvor genannten Legierungselemente auch als Kompensationselemente geeignet sind.

18. Verfahren nach einem der vorhergehenden Ansprüche, **dadurch gekennzeichnet, dass** die Analyse zumindest in der ersten Schmelze des letzten primärmetallurgischen Aggregates einer Erzeugungsroute, insbesondere ab einem Abstich von Rohstahl, vor oder nach einer ersten Zugabe von Legierungselementen und zumindest nach einer zweiten Zugabe von Legierungselementen durchgeführt wird.

19. Verfahren nach einem der vorhergehenden Ansprüche, **dadurch gekennzeichnet, dass**, wenn ein erstes Element durch einen Einsatzstoff in einem Anteil eingebracht wird, der unterhalb eines gewünschten, durch die Ziellegierung definierten Zielbereichs liegt, das erste Element, sofern es ein gewünschtes Legierungselement ist, in dem Umfang zulegiert wird, der zum Erreichen des Zielbereichs notwendig ist, oder, wenn das erste Element durch einen Einsatzstoff in einem Anteil eingebracht wird, der bereits im Zielbereich entspricht, das erste Element nicht mehr zulegiert wird oder, wenn das erste Element durch einen Einsatzstoff in einem Anteil eingebracht wird, der über dem Zielbereich liegt, das erste Element durch einen verringerten Legierungsanteil eines zweiten ähnlich wirkenden Elements oder einen erhöhten Legierungsanteil eines gegenläufig wirkenden dritten Elements kompensiert wird.

20. Verfahren nach einem der vorhergehenden Ansprüche, **dadurch gekennzeichnet, dass** das verwendete Korrekturmodell eine Reihung der unterschiedlichen Kompensationselemente nach auflösungs- und ausbringungsspezifischen sowie ökonomischen Gesichtspunkten festlegt.

21. Verfahren nach einem der vorhergehenden Ansprüche, **dadurch gekennzeichnet, dass** die Ziellegierung die folgende Ziellegierungszusammensetzung in Gew.-% umfasst:
| | |
|---|---|
| C | 0,05-1,3 |
| Si | 0,05-1,2 |
| Mn | 0,1-2,0 |
zudem Eisen und unvermeidbare Verunreinigungen,
wobei lediglich optional eines, mehrere oder alle der folgenden Elemente in Gew.-% enthalten sind:
| | |
|---|---|
| Cr | 0,001-2,0 |
| Cu | 0,001-0,4 |
| Mo | 0,001-1,0 |
| Ni | 0,001-2,5 |
| V | 0,001-0,4 |
| Sn | 0,001-0,03 |
| B | 0,0005-0,008 |
| N | 0,0001-0,02 |
| Ti | 0,001-0,04 |
| Al | 0,0005-0,05 |
| Nb | 0,001-0,1 |
| Co | 0,001-0,2 |
| Pb | 0,001-0,4 |
| W | 0,001-0,5 |
| P | ≤0,045 |
| S | ≤ 0,06. |

22. Steuersystem zur Herstellung von Stabstählen mit einem auf eine Stabstahlgüte spezifizierten Härteverlaufbereich, welcher durch eine Ziellegierung sichergestellt wird, und zur Durchführung des Verfahrens nach einem der vorhergehenden Ansprüche, **dadurch gekennzeichnet, dass** das Steuersystem zwei Komponenten umfasst: Ein Prognosemodell und ein Korrekturmodell, wobei das Prognosemodell dazu ausgelegt ist, eine Wirkung von Einflusselementen und Kompensationselementen auf den Härteverlauf des Stabstahls zu berechnen, und das Korrekturmodell dazu ausgelegt ist, Zielwerte von Kompensationselementen zu berechnen, um den Härteverlauf in einen Zielbereich zu bringen.

23. Steuersystem nach Anspruch 22, **dadurch gekennzeichnet, dass** zumindest drei Prognosemodelle und/oder Korrekturmodelle basierend auf durch die Ziellegierung definierten Filtern erzeugt werden, wobei ein erstes Prognosemodell und/oder Korrekturmodell für Stabstähle mit geringen Kohlenstoff- und Chromgehalten und insbesondere für eine Analyse umfassend C ≤ 0,2 Gew.-% und Cr ≤ 1,3 Gew.-% verwendet wird, wobei ein zweites Prognosemodell und/oder Korrekturmodell für Stabstähle mit höheren Kohlenstoff-, Chrom- und Mangangehalten und insbesondere für eine Analyse umfassend C ≤ 0,9 Gew.-%, 0,8 Gew.-% ≤ Cr ≤ 2,3 Gew.-% und Mn ≤ 1,5 Gew.-% verwendet wird und wobei ein drittes Prognosemodell und/oder Korrekturmodell für Stabstähle mit erhöhten Borgehalten und insbesondere für eine Analyse umfassend B > 0,0008 Gew.-% verwendet wird, wobei zusätzlich ein Borpotential B_{P} berücksichtigt wird.

24. Steuersystem nach Anspruch 22 oder 23, **dadurch gekennzeichnet, dass** zumindest ein weiteres Prognosemodell und/oder Korrekturmodell mittels der nachfolgenden Schritte erstellt wird:
i) Clusteranalyse mittels einer, mehrerer oder aller der folgenden Methoden: hierarchisches Clustering, partitionierendes Clustering, dichtebasierendes Clustering, gitterbasierendes Clustering;
ii) Bestimmung der optimalen Anzahl der Cluster mittels einer, mehrere oder aller der folgenden Methoden: Ellenbogenmethode, Silhouetten-Analyse, Entropiemethode, Intra-Cluster-Varianz, Gap-Statistik, Davies-Bouldin-Index, Calinski-Harabasz-Index, Akaike-Informationskriterium, Bayesianisches Informationskriterium, Hierarchische Clusteranalyse mit Dendrogramm, Gaussian Mixture Models;
iii) Optimierung, insbesondere mittels Methode kleinster Quadrate zur Bestimmung von Parametern Kₙ und k_{i,n} der multiplen linearen Regression, wobei Kₙ ein Grundhärtewert im Abstand n von der Oberfläche für eine Stabstahlgüte und k_{i,n} ein Gewichtungsfaktor, der den Einfluss eines bestimmten Elements auf die Härte im Abstand n von der Oberfläche beschreibt, ist.

25. Steuersystem nach Anspruch 23, **dadurch gekennzeichnet, dass** das Borpotential B_{P} zur Wirkung von Bor folgendermaßen bestimmbar ist: ${B}_{p} = Gew . - {%}_{B} - \frac{{M}_{B}}{{M}_{N}} \times \left(Gew.-{%}_{N}-\frac{{M}_{N}}{{M}_{Ti}}\times Gew.-{%}_{Ti}\right) ,$ wobei
Gew.-%ᵢ der Gehalt des jeweiligen Elements i und
Mᵢ die molare Masse des jeweiligen Elements i in g/mol ist.

26. Steuersystem nach einem der Ansprüche 22 bis 25, **dadurch gekennzeichnet, dass** zumindest ein positionsabhängiger Härtewert HRCₙ an einer bestimmten Position n innerhalb des Härteverlaufs mithilfe der folgenden Formel ermittelbar ist: ${HRC}_{n} = {K}_{n} + {\sum }_{i = 1}^{m} {k}_{i , n} ∗ Gew . - {%}_{i} ,$ wobei
n der Abstand des Härtewertes von der Oberfläche in mm bis in eine Tiefe von 50 mm,
Kₙ der Grundhärtewert im Abstand n von der Oberfläche,
k_{i,n} der Gewichtungsfaktor, der den Einfluss eines bestimmten Elements i auf die Härte im Abstand n von der Oberfläche wiedergibt,
m die Anzahl der berücksichtigten Elemente, und
Gew.-%ᵢ der Gehalt des jeweiligen Elements i ist.

27. Steuersystem nach einem der Ansprüche 22 bis 26, **dadurch gekennzeichnet, dass** die Ziellegierung die folgende Ziellegierungszusammensetzung in Gew.-% umfasst:
| | |
|---|---|
| C | 0,05-1,3 |
| Si | 0,05-1,2 |
| Mn | 0,1-2,0 |
zudem Eisen und unvermeidbare Verunreinigungen, wobei lediglich optional eines, mehrere oder alle der folgenden Elemente in Gew.-% enthalten sind:
| | |
|---|---|
| Cr | 0,001-2,0 |
| Cu | 0,001-0,4 |
| Mo | 0,001-1,0 |
| Ni | 0,001-2,5 |
| V | 0,001-0,4 |
| Sn | 0,001-0,03 |
| B | 0,0005-0,008 |
| N | 0,0001-0,02 |
| Ti | 0,001-0,04 |
| Al | 0,0005-0,05 |
| Nb | 0,001-0,1 |
| Co | 0,001-0,2 |
| Pb | 0,001-0,4 |
| W | 0,001-0,5 |
| P | ≤0,045 |
| S | ≤ 0,06. |

28. Stabstahl für Wärmebehandlungsanwendungen, hergestellt nach dem Verfahren nach einem der Ansprüche 1 bis 21.

## Geänderte Patentansprüche

### Geänderte Patentansprüche gemäss Regel 137(2) EPÜ.

1. Verfahren zur Herstellung von Stabstählen mit einem auf eine Stabstahlgüte spezifizierten Härteverlaufbereich, welcher durch eine Ziellegierung sichergestellt wird, wobei zumindest ein primärmetallurgisches Aggregat zu einer ersten Schmelze führt, wobei aus der ersten Schmelze zumindest eine Analyse von Gehalten der Elemente durchgeführt wird, wobei zumindest ein Ist-Wert der Schmelzanalyse an ein Steuersystem geleitet wird,
**dadurch gekennzeichnet, dass**
zwischen Einfluss- und Kompensationselementen unterschieden wird, wobei
Einflusselemente Elemente sind, welche einen Einfluss auf den Härteverlauf eines Endprodukts haben, und Kompensationselemente Elemente sind, die einen Einfluss auf den Härteverlauf haben und den Einfluss von Einflusselementen kompensieren, wobei
die Größe des Einflusses des zumindest einen Ist-Werts der jeweiligen Einfluss- und Kompensationselemente auf den Härteverlauf des Endprodukts bekannt ist, wobei
der zumindest eine Ist-Wert bezüglich derjenigen Einfluss- und Kompensationselemente erfasst wird, welche einen Einfluss auf den Härteverlauf des Endprodukts haben, wobei
zum Härteverlauf des Endprodukts zumindest ein Kompensationselement ermittelt wird, welches den Härteverlauf einstellt, wobei
eines oder mehrere Kompensationselemente in die Schmelze in einer Menge zulegiert werden, welche den Härteverlauf in einen Zielbereich bringen und den Einfluss eines oder mehrerer Einflusselemente auf den Härteverlauf kompensieren, und wobei
hierfür im Steuersystem zwei Komponenten verwendet werden: ein Prognosemodell und ein Korrekturmodell, wobei mit dem Prognosemodell der Einfluss der Einfluss- und Kompensationselemente auf den Härteverlauf des Endprodukts und mit dem Korrekturmodell korrigierte Zielbereiche der Kompensationselemente berechnet werden, um den Härteverlauf in einen Zielbereich zu bringen, wobei zumindest ein Prognosemodell und Korrekturmodell unter Anwendung einer multiplen linearen Regression für einen stabilen Härteverlauf erzeugt wird, wobei
für die Erstellung des zumindest einen Prognosemodells und/oder Korrekturmodells zunächst ein Trainingsdatensatz basierend auf einer Ermittlung der Härtbarkeit von Stahl durch EN ISO 642 erstellt wird und hierauf basierend zumindest ein Prognosemodell und/oder Korrekturmodell für die spezifizierte Stabstahlgüte erzeugt wird, wobei
bei der Erzeugung des zumindest einen Prognosemodells und/oder Korrekturmodells als Elemente eines, mehrere oder alle aus der Gruppe von C, Si, Mn, P, S, Cu, Cr, Ni, Mo, V, Ti, Al, B, Nb, W, N, Co und Pb berücksichtigt werden, und wobei
die Einflusselemente eines, mehrere oder alle Elemente aus der Gruppe Cu, Mo, Sn, Ni umfassen und die übrigen Elemente Kompensationselemente sind.

2. Verfahren nach Anspruch 1, **dadurch gekennzeichnet, dass** der Härteverlaufbereich nach EN ISO 683 auf die Stabstahlgüte spezifiziert wird.

3. Verfahren nach Anspruch 1 oder 2, **dadurch gekennzeichnet, dass** die Analyse mittels Probenentnahme nach EN ISO 14284 durchgeführt wird.

4. Verfahren nach einem der vorhergehenden Ansprüche, **dadurch gekennzeichnet, dass** auf die Stahlherstellung folgende Nachbehandlungsschritte bis zum Erhalt des Endprodukts unverändert durchgeführt werden.

5. Verfahren nach einem der vorhergehenden Ansprüche, **dadurch gekennzeichnet, dass** zumindest drei Prognosemodelle und/oder Korrekturmodelle basierend auf durch die Ziellegierung definierten Filtern erzeugt werden, wobei ein erstes Prognosemodell und/oder Korrekturmodell für Stabstähle mit geringen Kohlenstoff- und Chromgehalten und insbesondere für eine Analyse umfassend C ≤ 0,2 Gew.-% und Cr ≤ 1,3 Gew.-% verwendet wird, wobei ein zweites Prognosemodell und/oder Korrekturmodell für Stabstähle mit höheren Kohlenstoff-, Chrom- und Mangangehalten und insbesondere für eine Analyse umfassend C ≤ 0,9 Gew.-%, 0,8 Gew.-% ≤ Cr ≤ 2,3 Gew.-% und Mn ≤ 1,5 Gew.-% verwendet wird und wobei ein drittes Prognosemodell und/oder Korrekturmodell für Stabstähle mit erhöhten Borgehalten und insbesondere für eine Analyse umfassend B > 0,0008 Gew.-% verwendet wird, wobei zusätzlich ein Borpotential B_{P} berücksichtigt wird.

6. Verfahren nach einem der vorhergehenden Ansprüche, **dadurch gekennzeichnet, dass** der zumindest eine erfasste Ist-Wert der Analyse unter Heranziehung des Mittelwerts und/oder der Summe der Quadratwurzeln der mittleren quadratischen Abweichungen von positionsabhängigen Härtewerten HRCₙ dem zumindest einen Prognosemodell und/oder Korrekturmodell zugeordnet wird, wobei für den zumindest einen Ist-Wert der Mittelwert und/oder die Summe der Quadratwurzeln der mittleren quadratischen Abweichungen der positionsabhängigen Härtewerte HRCₙ für alle Prognosemodelle und/oder Korrekturmodelle ermittelt wird und basierend auf den Ergebnissen jenes Prognosemodell und/oder Korrekturmodell mit der höchsten Genauigkeit gewählt wird.

7. Verfahren nach einem der vorhergehenden Ansprüche, **dadurch gekennzeichnet, dass** zumindest ein weiteres Prognosemodell und/oder Korrekturmodell mittels der nachfolgenden Schritte erstellt wird:
i) Clusteranalyse mittels einer, mehrerer oder aller der folgenden Methoden: hierarchisches Clustering, partitionierendes Clustering, dichtebasierendes Clustering, gitterbasierendes Clustering;
ii) Bestimmung der optimalen Anzahl der Cluster mittels einer, mehrere oder aller der folgenden Methoden: Ellenbogenmethode, Silhouetten-Analyse, Entropiemethode, Intra-Cluster-Varianz, Gap-Statistik, Davies-Bouldin-Index, Calinski-Harabasz-Index, Akaike-Informationskriterium, Bayesianisches Informationskriterium, Hierarchische Clusteranalyse mit Dendrogramm, Gaussian Mixture Models;
iii) Optimierung, insbesondere mittels Methode kleinster Quadrate zur Bestimmung von Parametern Kₙ und k_{i,n} der multiplen linearen Regression, wobei Kₙ ein Grundhärtewert für eine Stabstahlgüte im Abstand n von der Oberfläche und k_{i,n} ein Gewichtungsfaktor, der den Einfluss eines bestimmten Elements auf die Härte im Abstand n von der Oberfläche beschreibt, ist.

8. Verfahren nach Anspruch 7, **dadurch gekennzeichnet, dass** die Clusteranalyse zentroidbasiert ist und mittels nicht hierarchischem, partitionierendem Clustering, insbesondere K-Means-Methode durchgeführt wird.

9. Verfahren nach Anspruch 7, **dadurch gekennzeichnet, dass** die Bestimmung der optimalen Anzahl an Cluster mittels der Silhouetten- oder Ellenbogenmethode, insbesondere der Silhouetten-Methode durchgeführt wird.

10. Verfahren nach Anspruch 8, **dadurch gekennzeichnet, dass** im Rahmen der K-Means-Methode ein Proximitätsmaß zur Kategorisierung der Objekte hinsichtlich eines Merkmals Distanz oder Ähnlichkeit, insbesondere des Merkmals Distanz verwendet wird.

11. Verfahren nach Anspruch 10, **dadurch gekennzeichnet, dass** bei metrischen Variablen eine euklidische Distanz, quadrierte euklidische Distanz oder L1-Distanz, insbesondere die euklidische Distanz verwendet wird.

12. Verfahren nach Anspruch 5, **dadurch gekennzeichnet, dass** das Borpotential B_{P} zur Wirkung von Bor folgendermaßen bestimmt wird: ${B}_{p} = Gew . - {%}_{B} - \frac{{M}_{B}}{{M}_{N}} \times \left(Gew.-{%}_{N}-\frac{{M}_{N}}{{M}_{Ti}}\times Gew.-{%}_{Ti}\right) ,$ wobei
Gew.-%i der Gehalt des jeweiligen Elements i und
Mᵢ die molare Masse des jeweiligen Elements i in g/mol ist.

13. Verfahren nach Anspruch 7, **dadurch gekennzeichnet, dass** unter Heranziehung des zumindest einen Prognosemodells und/oder Korrekturmodells die Parameter Kₙ und k_{i,n} als Ausgangspunkte gewählt werden.

14. Verfahren nach einem der vorhergehenden Ansprüche, **dadurch gekennzeichnet, dass** zumindest ein positionsabhängiger Härtewert HRCₙ an einer bestimmten Position n innerhalb des Härteverlaufs mithilfe der folgenden Formel ermittelt wird: ${HRC}_{n} = {K}_{n} + {\sum }_{i = 1}^{m} {k}_{i , n} ∗ Gew . - {%}_{i} ,$ wobei
n der Abstand des Härtewertes von der Oberfläche in mm bis in eine Tiefe von 50 mm,
Kₙ der Grundhärtewert im Abstand n von der Oberfläche,
k_{i,n} der Gewichtungsfaktor, der den Einfluss eines bestimmten Elements i auf die Härte im Abstand n von der Oberfläche wiedergibt,
m die Anzahl der berücksichtigten Elemente, und
Gew.-%ᵢ der Gehalt des jeweiligen Elements i ist,
wobei bei der Ermittlung des Härtewerts HRCₙ als Elemente eines, mehrere oder alle aus der Gruppe von C, Si, Mn, P, S, Cu, Cr, Ni, Mo, V, Ti, Al, B, Nb, W, N, Co und Pb berücksichtigt werden.

15. Verfahren nach einem der vorhergehenden Ansprüche, **dadurch gekennzeichnet, dass** bei Ni-legierten Stabstahlgüten Cu, Mo, Sn, bei Mo-legierten Stabstahlgüten Cu, Sn, Ni und bei Ni-Mo-legierten Stabstahlgüten Cu, Sn Einflusselemente sind, wobei in diesen Fällen entsprechend die zuvor genannten Legierungselemente auch als Kompensationselemente geeignet sind.

16. Verfahren nach einem der vorhergehenden Ansprüche, **dadurch gekennzeichnet, dass** die Analyse zumindest in der ersten Schmelze des letzten primärmetallurgischen Aggregates einer Erzeugungsroute, insbesondere ab einem Abstich von Rohstahl, vor oder nach einer ersten Zugabe von Legierungselementen und zumindest nach einer zweiten Zugabe von Legierungselementen durchgeführt wird.

17. Verfahren nach einem der vorhergehenden Ansprüche, **dadurch gekennzeichnet, dass**, wenn ein erstes Element durch einen Einsatzstoff in einem Anteil eingebracht wird, der unterhalb eines gewünschten, durch die Ziellegierung definierten Zielbereichs liegt, das erste Element, sofern es ein gewünschtes Legierungselement ist, in dem Umfang zulegiert wird, der zum Erreichen des Zielbereichs notwendig ist, oder, wenn das erste Element durch einen Einsatzstoff in einem Anteil eingebracht wird, der bereits im Zielbereich entspricht, das erste Element nicht mehr zulegiert wird oder, wenn das erste Element durch einen Einsatzstoff in einem Anteil eingebracht wird, der über dem Zielbereich liegt, das erste Element durch einen verringerten Legierungsanteil eines zweiten ähnlich wirkenden Elements oder einen erhöhten Legierungsanteil eines gegenläufig wirkenden dritten Elements kompensiert wird.

18. Verfahren nach einem der vorhergehenden Ansprüche, **dadurch gekennzeichnet, dass** das verwendete Korrekturmodell eine Reihung der unterschiedlichen Kompensationselemente nach auflösungs- und ausbringungsspezifischen sowie ökonomischen Gesichtspunkten festlegt.

19. Verfahren nach einem der vorhergehenden Ansprüche, **dadurch gekennzeichnet, dass** die Ziellegierung die folgende Ziellegierungszusammensetzung in Gew.-% umfasst:
| | |
|---|---|
| C | 0,05-1,3 |
| Si | 0,05-1,2 |
| Mn | 0,1-2,0 |
zudem Eisen und unvermeidbare Verunreinigungen,
wobei lediglich optional eines, mehrere oder alle der folgenden Elemente in Gew.-% enthalten sind:
| | |
|---|---|
| Cr | 0,001-2,0 |
| Cu | 0,001-0,4 |
| Mo | 0,001-1,0 |
| Ni | 0,001-2,5 |
| V | 0,001-0,4 |
| Sn | 0,001-0,03 |
| B | 0,0005-0,008 |
| N | 0,0001-0,02 |
| Ti | 0,001-0,04 |
| Al | 0,0005-0,05 |
| Nb | 0,001-0,1 |
| Co | 0,001-0,2 |
| Pb | 0,001-0,4 |
| W | 0,001-0,5 |
| P | ≤0,045 |
| S | ≤ 0,06. |

20. Steuersystem zur Herstellung von Stabstählen mit einem auf eine Stabstahlgüte spezifizierten Härteverlaufbereich, welcher durch eine Ziellegierung sichergestellt wird, und zur Durchführung des Verfahrens nach einem der vorhergehenden Ansprüche, **dadurch gekennzeichnet, dass** das Steuersystem zwei Komponenten umfasst: Ein Prognosemodell und ein Korrekturmodell, wobei das Prognosemodell dazu ausgelegt ist, eine Wirkung von Einflusselementen und Kompensationselementen auf den Härteverlauf des Stabstahls zu berechnen, und das Korrekturmodell dazu ausgelegt ist, Zielwerte von Kompensationselementen zu berechnen, um durch Zulegieren zumindest eines Kompensationselements den Härteverlauf in einen Zielbereich zu bringen.

21. Steuersystem nach Anspruch 20, **dadurch gekennzeichnet, dass** zumindest drei Prognosemodelle und/oder Korrekturmodelle basierend auf durch die Ziellegierung definierten Filtern erzeugt werden, wobei ein erstes Prognosemodell und/oder Korrekturmodell für Stabstähle mit geringen Kohlenstoff- und Chromgehalten und insbesondere für eine Analyse umfassend C ≤ 0,2 Gew.-% und Cr ≤ 1,3 Gew.-% verwendet wird, wobei ein zweites Prognosemodell und/oder Korrekturmodell für Stabstähle mit höheren Kohlenstoff-, Chrom- und Mangangehalten und insbesondere für eine Analyse umfassend C ≤ 0,9 Gew.-%, 0,8 Gew.-% ≤ Cr ≤ 2,3 Gew.-% und Mn ≤ 1,5 Gew.-% verwendet wird und wobei ein drittes Prognosemodell und/oder Korrekturmodell für Stabstähle mit erhöhten Borgehalten und insbesondere für eine Analyse umfassend B > 0,0008 Gew.-% verwendet wird, wobei zusätzlich ein Borpotential B_{P} berücksichtigt wird.

22. Steuersystem nach Anspruch 20 oder 21, **dadurch gekennzeichnet, dass** zumindest ein weiteres Prognosemodell und/oder Korrekturmodell mittels der nachfolgenden Schritte erstellt wird:
i) Clusteranalyse mittels einer, mehrerer oder aller der folgenden Methoden: hierarchisches Clustering, partitionierendes Clustering, dichtebasierendes Clustering, gitterbasierendes Clustering;
ii) Bestimmung der optimalen Anzahl der Cluster mittels einer, mehrere oder aller der folgenden Methoden: Ellenbogenmethode, Silhouetten-Analyse, Entropiemethode, Intra-Cluster-Varianz, Gap-Statistik, Davies-Bouldin-Index, Calinski-Harabasz-Index, Akaike-Informationskriterium, Bayesianisches Informationskriterium, Hierarchische Clusteranalyse mit Dendrogramm, Gaussian Mixture Models;
iii) Optimierung, insbesondere mittels Methode kleinster Quadrate zur Bestimmung von Parametern Kₙ und k_{i,n} der multiplen linearen Regression, wobei Kₙ ein Grundhärtewert im Abstand n von der Oberfläche für eine Stabstahlgüte und k_{i,n} ein Gewichtungsfaktor, der den Einfluss eines bestimmten Elements auf die Härte im Abstand n von der Oberfläche beschreibt, ist.

23. Steuersystem nach Anspruch 21, **dadurch gekennzeichnet, dass** das Borpotential B_{P} zur Wirkung von Bor folgendermaßen bestimmbar ist: ${B}_{p} = Gew . - {%}_{B} - \frac{{M}_{B}}{{M}_{N}} \times \left(Gew.-{%}_{N}-\frac{{M}_{N}}{{M}_{Ti}}\times Gew.-{%}_{Ti}\right) ,$ wobei
Gew.-%ᵢ der Gehalt des jeweiligen Elements i und
Mᵢ die molare Masse des jeweiligen Elements i in g/mol ist.

24. Steuersystem nach einem der Ansprüche 20 bis 23, **dadurch gekennzeichnet, dass** zumindest ein positionsabhängiger Härtewert HRCₙ an einer bestimmten Position n innerhalb des Härteverlaufs mithilfe der folgenden Formel ermittelbar ist: ${HRC}_{n} = {K}_{n} + {\sum }_{i = 1}^{m} {k}_{i , n} ∗ Gew . - {%}_{i} ,$ wobei
n der Abstand des Härtewertes von der Oberfläche in mm bis in eine Tiefe von 50 mm,
Kₙ der Grundhärtewert im Abstand n von der Oberfläche,
k_{i,n} der Gewichtungsfaktor, der den Einfluss eines bestimmten Elements i auf die Härte im Abstand n von der Oberfläche wiedergibt,
m die Anzahl der berücksichtigten Elemente, und
Gew.-%i der Gehalt des jeweiligen Elements i ist.

25. Steuersystem nach einem der Ansprüche 20 bis 24, **dadurch gekennzeichnet, dass** die Ziellegierung die folgende Ziellegierungszusammensetzung in Gew.-% umfasst:
| | |
|---|---|
| C | 0,05-1,3 |
| Si | 0,05-1,2 |
| Mn | 0,1-2,0 |
zudem Eisen und unvermeidbare Verunreinigungen,
wobei lediglich optional eines, mehrere oder alle der folgenden Elemente in Gew.-% enthalten sind:
| | |
|---|---|
| Cr | 0,001-2,0 |
| Cu | 0,001-0,4 |
| Mo | 0,001-1,0 |
| Ni | 0,001-2,5 |
| V | 0,001-0,4 |
| Sn | 0,001-0,03 |
| B | 0,0005-0,008 |
| N | 0,0001-0,02 |
| Ti | 0,001-0,04 |
| Al | 0,0005-0,05 |
| Nb | 0,001-0,1 |
| Co | 0,001-0,2 |
| Pb | 0,001-0,4 |
| W | 0,001-0,5 |
| P | ≤0,045 |
| S | ≤ 0,06. |

26. Stabstahl für Wärmebehandlungsanwendungen, hergestellt nach dem Verfahren nach einem der Ansprüche 1 bis 19.
